# EUROPEAN PATENT APPLICATION

(11) **EP 3 896 081 A1**
(43) Date of publication of application: **20.10.2021**
(21) Application number: 19895947.0
(22) Date of filing: 12.12.2019
(51) Int. Cl.: C07K 14/78, C12P 21/06, A61L 27/24

(54) **COLLAGEN SOLID, METHOD FOR PRODUCING COLLAGEN SOLID, BIOMATERIAL, AND EX VIVO MATERIAL**

(30) Priority: 12.12.2018 JP 2018232811; 22.04.2019 JP 2019081130
(71) Applicant: Kinki University, Higashi-Osaka-shi Osaka 577-8502 (JP)
(72) Inventor: MORIMOTO, Koichi, Kinokawa-shi, Wakayama 649-6493 (JP); KUNII, Saori, Kinokawa-shi, Wakayama 649-6493 (JP); FUKASE, Naomasa, Kobe-shi, Hyogo 657-8501 (JP); KURODA, Ryosuke, Kobe-shi, Hyogo 657-8501 (JP); TAKEMORI, Toshiyuki, Kobe-shi, Hyogo 657-8501 (JP)
(74) Representative: Bittner, Thomas L.
(86) International application number: PCT/JP2019/048788
(87) International publication number: WO 2020/122198

(57) **Abstract**

The present invention provides a collagen solid having higher strength and density. A collagen solid is used which contains a collagen-cysteine protease degradation product or an atelocol-lagen-cysteine protease degradation product and has a density of 50 mg/cm³ or more. (Fig. 1)

## Description

### Technical Field

The present invention relates to a collagen solid, a method for producing the collagen solid, a biomaterial, and an ex vivo material.

### Background Art

Collagen, which is a protein constituting a connective tissue between cells or a bone tissue of an animal, or gelatin, which is a thermally denatured material of collagen, has been conventionally used in various applications.

If a bone is defective, complete self-renewal is difficult. Therefore, bone regeneration therapy using an autologous bone or a bone derived from a conspecific organism has been conventionally carried out. For transplantation of an autologous bone, it is necessary to extract a portion of one's own bone, and there is a limit to an amount of bone that can be extracted from our body. Meanwhile, for transplantation of a bone derived from a conspecific organism, a bone of another person is used. Therefore, a risk of infection is high. Under the circumstances, in recent years, bone regeneration therapy has been carried out using biomaterials such as hydroxyapatite or β-TCP. However, the biomaterials themselves described above do not have an ability to create a new bone, and a rate of bone formation is significantly inferior to that of an autologous bone. In recent years, bone regeneration therapy has been carried out using stem cells and scaffolds formed of biomaterials. However, there are many problems in this technique, such as the need for a cell culture facility and a high cost. Under such circumstances, development of a new technique for bone regeneration therapy is currently demanded.

Collagen, which is a protein constituting a connective tissue between cells or a bone tissue of an animal, is conventionally used as a biomaterial to be implanted in a living body (e.g., as a biomaterial to complement a defective or damaged biological tissue).

Meanwhile, collagen can form a scaffold for cells to adhere to a substrate. Therefore, conventionally, a technique of forming a scaffold by coating a substrate with an aqueous solution containing collagen has been used.

For example, Patent Literature 1 discloses a composite type of bone filling material composed of a combination of collagen and a calcium phosphate compound. Patent Literature 2 discloses a biomaterial containing collagen, calcium phosphate and sugar as main components. Non-patent Literature 1 discloses a biomaterial composed of collagen crosslinked by glutaraldehyde.

In addition, Patent Literature 3 discloses a degradation product of collagen or atelocollagen, a method for producing the degradation product, and use of the degradation product. The degradation product has spheroid-forming activity. Meanwhile, Patent Literature 4 discloses a differentiation-inducing composition containing a degradation product of collagen or atelocollagen. The differentiation-inducing composition has spheroid-forming activity, bone differentiation inducing ability, and the like.

In addition, in the field of carrying out cell culture, coating a substrate with an aqueous solution containing collagen is widely conducted.

### Citation List

[Patent Literature]
[Patent Literature 1]
   Japanese Patent Application Publication Tokukai No. 2000-262608
[Patent Literature 2]
   Japanese Patent Application Publication Tokukai No. 2009-5814
[Patent Literature 3]
   WO2015/167003
[Patent Literature 4]
   WO2015/167004

### [Non-patent Literature]

L. H. H. Olde Demink et. al., Journal of Materials science: Materials in Medicine: 6, pp. 460-472, 1995

### Summary of Invention

### Technical Problem

Sites in a living body where a biomaterial is to be implanted often have complex shapes. In order for the biomaterial to function well in the living body, it is important to adapt a shape of the biomaterial to a shape of a site at which the biomaterial is to be implanted. However, the conventional biomaterial has a problem that the biomaterial does not have high strength (in other words, the conventional biomaterial is soft) and therefore cannot be processed into an intended shape.

Moreover, the technique of coating a substrate with an aqueous solution containing collagen has a problem that a high concentration of collagen, which is comparable to a collagen concentration in a living body, cannot be adsorbed on a substrate surface.

The degradation product disclosed in Patent Literature 3 is in the form of solution. Patent Literature 3 does not disclose a concept of concentrating the degradation product into a highly dense solid. Of course, Patent Literature 3 does not disclose a tangent modulus of the solid. Further, the differentiation-inducing composition disclosed in Patent Literature 4 is also in the form of solution. Patent Literature 4 does not disclose a concept of concentrating the degradation product into a highly dense solid. Of course, Patent Literature 4 does not disclose a tangent modulus of the solid.

As described above, both of the degradation product disclosed in Patent Literature 3 and the differentiation-inducing composition disclosed in Patent Literature 4 are in the form of solution. The degradation product and the differentiation-inducing composition which are implanted into living bodies easily diffuse and are lost from the implanted sites. As such, there has been room for further improvement in regeneration of a living tissue (e.g., bone) at a target site. Moreover, even if the substrate is coated with the degradation product or the differentiation-inducing composition, it is not possible to adsorb a high concentration of collagen, which is comparable to the collagen concentration in a living body, on the substrate surface. There has been room for further improvement in adsorption of a high concentration of collagen, which is comparable to the collagen concentration in a living body, on the substrate surface.

In addition, a bone (e.g., femur) is a biological tissue to which a large load is to be applied and, when a biomaterial is implanted into a bone or the like, it is demanded that the biomaterial itself has high strength. Moreover, there is a possibility that a large load is to be applied to a substrate or the like used in cell culture, and/or the substrate is to be used in long-term culture. Therefore, the substrate or the like needs to have high strength. However, the conventional material has a problem that the material does not have high strength (in other words, the conventional material is soft).

In order to achieve high strength in a conventional material, it is necessary to employ an auxiliary material different from collagen (e.g., a crosslinking agent or a synthetic polymer). However, the auxiliary material has the following problems: (i) the auxiliary material increases a cost of material and/or increases immunogenicity, inflammation, retention, and the like of the material in a living body, thereby reducing safety; and (ii) the auxiliary material adversely affects cells to be cultured and/or makes it impossible to culture cells under the same conditions as in the living body.

In addition, conventional materials have a problem that it is difficult to incorporate an optional substance component into the material because, for example, solubility of the raw material is low or strength of the material is low.

An object of the present invention is to provide a collagen solid which has a high density and high strength.

### Solution to Problem

In a case where collagen is degraded using a protease such as pepsin, merely a solution is obtained which contains a large amount of insoluble precipitate and soluble impurities and contains a soluble degradation product at a low concentration (i.e., 20 mg/mL or less as shown in Example described later).

The inventors of the present invention have found the following facts: (i) by degrading collagen or atelocollagen using a cysteine protease, it is possible to obtain a solution containing a solubilized degradation product at a high concentration (i.e., 30 mg/mL or more as shown in Example described later); (ii) by removing a solvent from the solution, it is possible to obtain a collagen solid (hereinafter referred to as "LASCol", which is an abbreviation of low adhesive scaffold collagen) which contains the collagen degradation product at a high density (i.e., 50 mg/cm³ or more as shown in Example described later), and/or has a large tangent modulus (i.e., 90 kPa or more as shown in Example described later), and consequently can be processed into an intended shape; and (iii) the collagen solid (LASCol) is prepared from collagen that is originally present in a living body, and therefore can be used safely and reproducibly as a biomaterial for bone regeneration and a biomaterial for cell culture. On the basis of those findings, the inventors have accomplished the present invention.
[1] In order to attain the object, a collagen solid in accordance with an aspect of the present invention contains a collagen-cysteine protease degradation product or an atelocollagen-cysteine protease degradation product, the collagen solid having a density of 50 mg/cm³ or more.
[2] The collagen solid in accordance with an aspect of the present invention for attaining the object has a tangent modulus of 90 kPa or more.
[3] The collagen solid in accordance with an aspect of the present invention further contains an optional substance.
[4] In order to attain the object, a biomaterial in accordance with an aspect of the present invention contains the collagen solid described in any one of [1] through [3].
[5] In order to attain the object, an ex vivo material in accordance with an aspect of the present invention contains the collagen solid described in any one of [1] through [3].
[6] In order to attain the object, a biomaterial in accordance with an aspect of the present invention is a bone regeneration material containing the collagen solid described in any one of [1] through [3].
[7] In order to attain the object, a method for producing a collagen solid in accordance with an aspect of the present invention is a method for producing the collagen solid described in any one of [1] through [3] and includes: a degradation step of degrading collagen or atelocollagen with a cysteine protease; and a removal step of removing a solvent from a collagen degradation product or an atelocollagen degradation product which has been obtained in the degradation step.
[8] In the method for producing a collagen solid in accordance with an aspect of the present invention, in the removal step, an optional substance is added to the collagen degradation product or the atelocollagen degradation product which has been obtained in the degradation step to obtain a mixture, and then the solvent is removed from the mixture.
[9] In the method for producing a collagen solid in accordance with an aspect of the present invention, in the removal step, a collagen solid which has been obtained in the removal step is caused to adsorb an optional substance.

### Advantageous Effects of Invention

According to an aspect of the present invention, it is possible to provide a collagen solid having a higher density and higher strength. Further, according to an aspect of the present invention, it is possible to provide a collagen solid which can be easily processed into an intended shape. Further, according to an aspect of the present invention, it is possible to provide a novel biomaterial or ex vivo material containing a collagen solid having a higher density and higher strength. Further, according to an aspect of the present invention, it is possible to provide a collagen solid which contains one or more optional substances in an arbitrary amount, and to provide a novel biomaterial or ex vivo material containing such a collagen solid. Further, according to an aspect of the present invention, it is possible to provide a biomaterial (in other words, bone regeneration material) which can regenerate or repair a biological tissue (e.g., bone). Further, according to an aspect of the present invention, it is possible to provide an ex vivo material (in other words, material for cell culture) which can be used in cell culture.

### Brief Description of Drawings

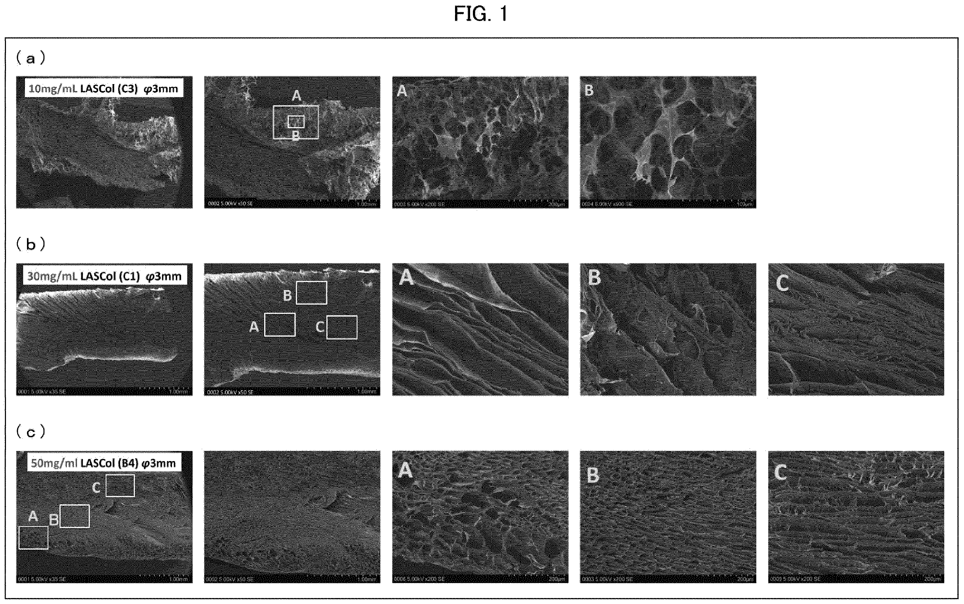

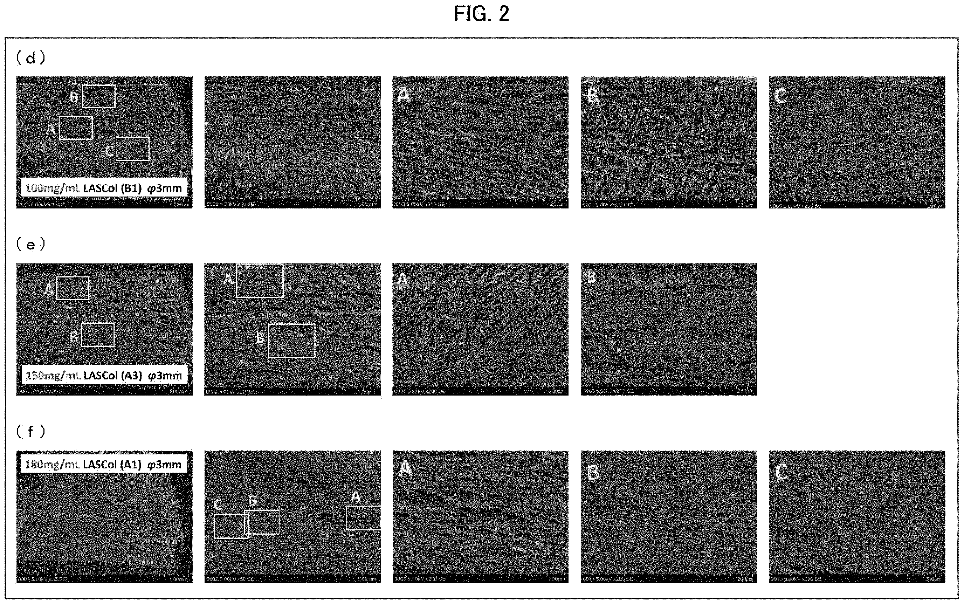

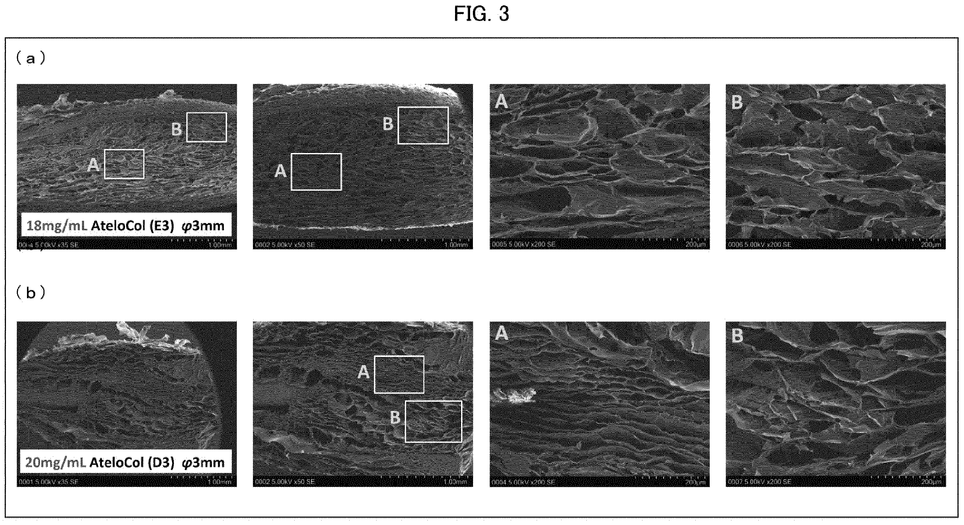

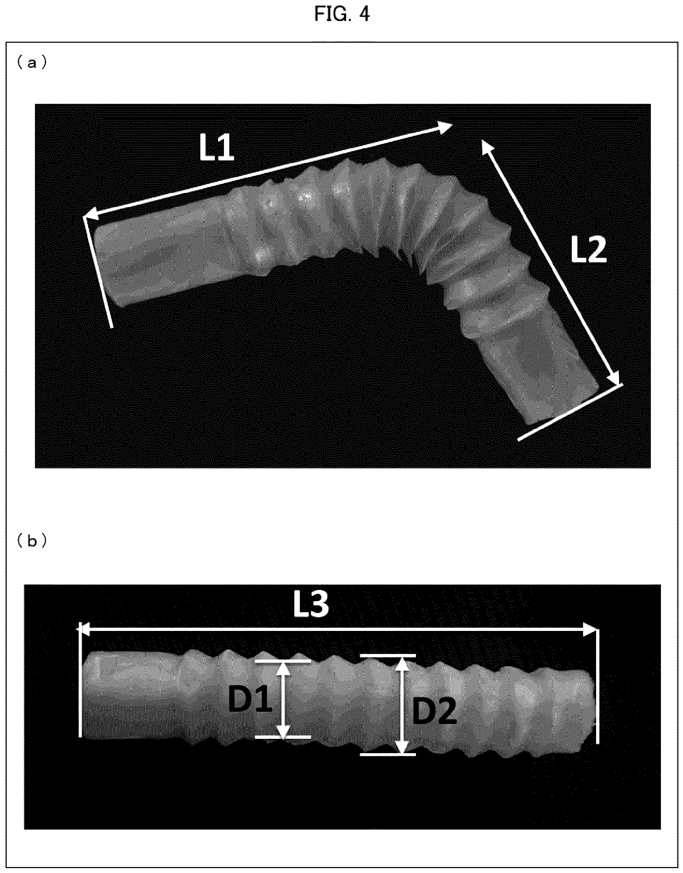

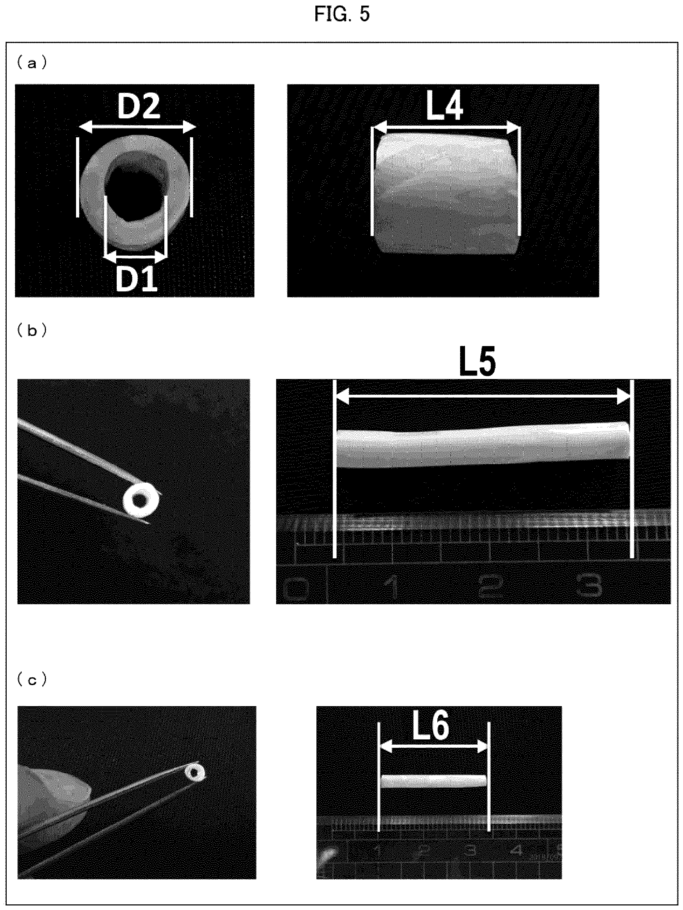

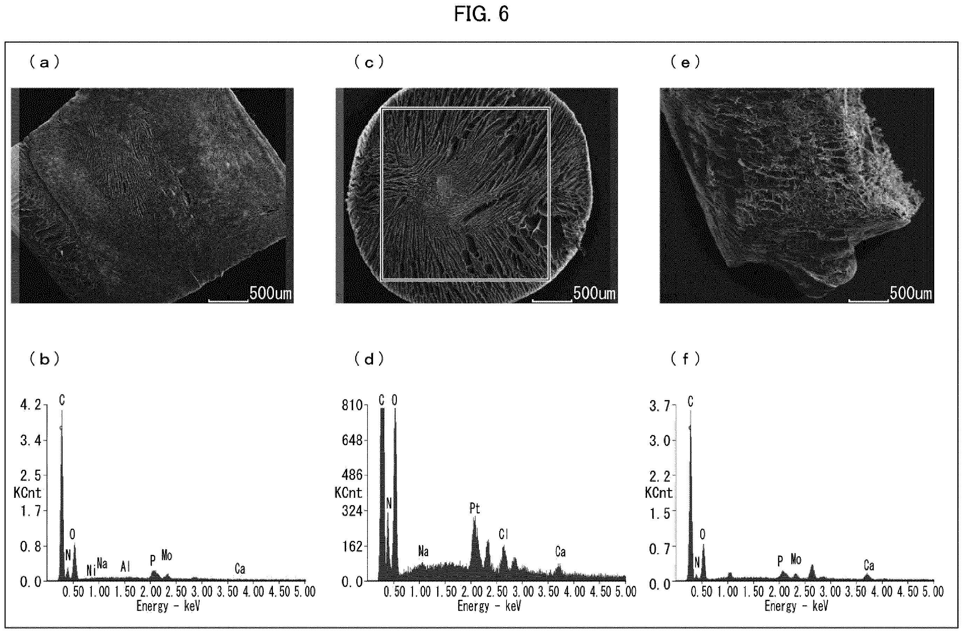

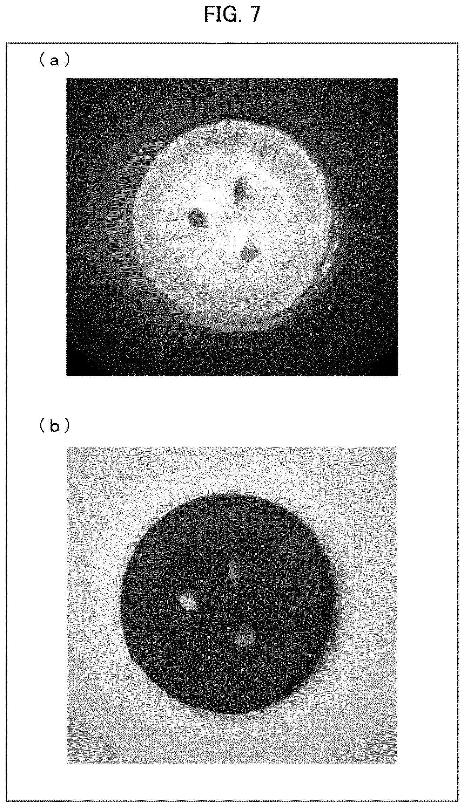

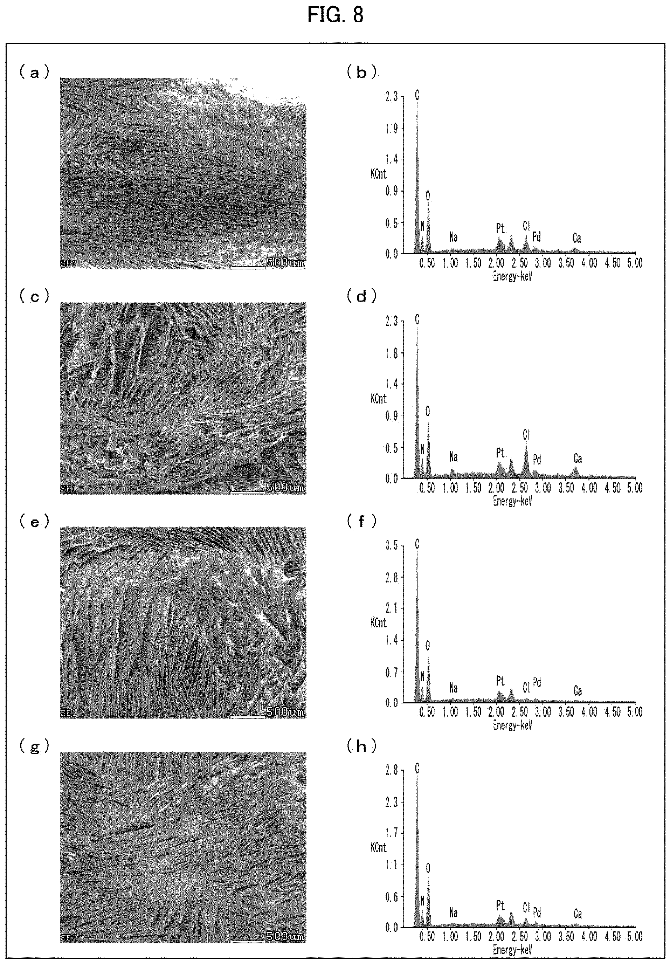

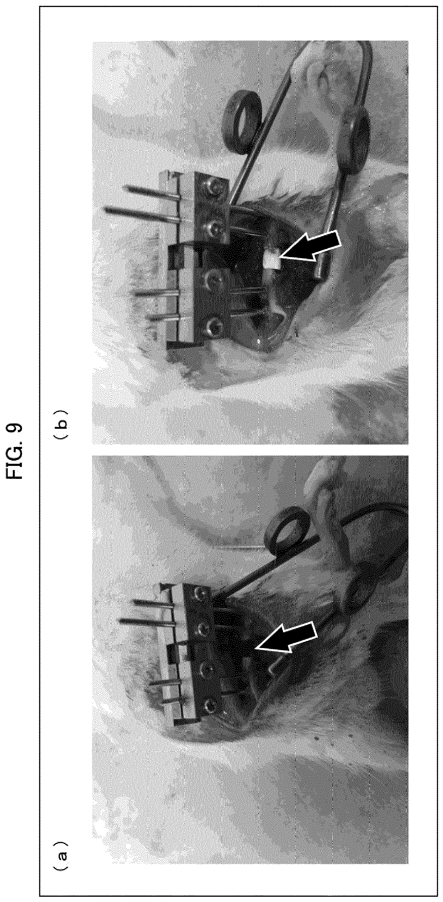

- (a) through (c) of Fig. 1: show cross-sectional images of columnar collagen solids in accordance with Example of the present invention.
- (d) through (f) of Fig. 2: show cross-sectional images of columnar collagen solids in accordance with Example of the present invention.
- (a) and (b) of Fig. 3: show cross-sectional images of columnar atelocollagen solids in accordance with Comparative Example of the present invention.
- (a) of Fig. 4: is an image showing a bellows-shaped collagen solid in a bent state, and
- (b) of Fig. 4: is an image showing the bellows-shaped collagen solid in a stretched state.
- (a) through (c) of Fig. 5: show images of small-diameter-tubular collagen solids in accordance with Example of the present invention.
- (a) through (f) of Fig. 6: show SEM images and results of SEM-EDX analysis of columnar collagen solids in accordance with Examples of the present invention.
- (a) and (b) of Fig. 7: show images of a columnar collagen solid having small holes in accordance with Example of the present invention.
- (a) through (h) of Fig. 8: show SEM images and results of SEM-EDX analysis of collagen solids in accordance with Example of the present invention.
- (a) and (b) of Fig. 9: are images showing processes for preparing a 4 mm femur defect rat model in accordance with Example of the present invention.

- Fig. 10: is a view showing evaluation criteria of the modified RUST score used in evaluation with medical imaging technology in accordance with Example of the present invention.
- Fig. 11: shows radiographic images of bones of a 1 mm femur defect population, a 50 mg/mL LASCol solid implanted population, a 100 mg/mL LASCol solid implanted population, and a 150 mg/mL LASCol solid implanted population taken immediately after implantation, 14 days after implantation, and 28 days after implantation, in accordance with Example of the present invention.
- Fig. 12: is a graph showing results of evaluating bone adhesion on the 28th day after implantation based on the modified RUST score in the 1 mm femur defect population, the 50 mg/mL LASCol solid implanted population, the 100 mg/mL LASCol solid implanted population, and the 150 mg/mL LASCol solid implanted population in accordance with Example of the present invention.
- Fig. 13: shows µCT images of the 1 mm femur defect population, the 50 mg/mL LASCol solid implanted population, the 100 mg/mL LASCol solid implanted population, and the 150 mg/mL LASCol solid implanted population taken 28 days after implantation, in accordance with Example of the present invention.
- Fig. 14: shows µCT images of the 100 mg/mL LASCol solid implanted population taken 28 days after implantation, in accordance with Example of the present invention.
- Fig. 15: shows µCT images of the 150 mg/mL LASCol solid implanted population taken 14 days after implantation, in accordance with Example of the present invention.
- Fig. 16: shows µCT images of the 150 mg/mL LASCol solid implanted population taken 28 days after implantation, in accordance with Example of the present invention.
- Fig. 17: is an image showing a femur extracted from a rat in the 150 mg/mL LASCol solid implanted population taken 28 days after implantation, in accordance with Example of the present invention.
- Fig. 18: is a view showing histological evaluation criteria based on the Allen's score, in accordance with Example of the present invention.
- Fig. 19: shows HE stained images of sectioned femur tissues of a 1 mm femur defect individual and a 50 mg/mL LASCol solid implanted individual taken 14 days after implantation and 28 days after implantation, in accordance with Example of the present invention.
- Fig. 20: shows SO stained images of the same sectioned tissues as those in Fig. 19 (i.e., the sectioned femur tissues of a 1 mm femur defect individual and a 50 mg/mL LASCol solid implanted individual on the 14th day after implantation and 28th day after implantation) and evaluation results based on the Allen's score, in accordance with Example of the present invention.
- Fig. 21: shows SO stained images of sectioned femur tissues of a 100 mg/mL LASCol solid implanted population taken 28 days after implantation and evaluation results based on the Allen's score, in accordance with Example of the present invention.
- Fig. 22: shows SO stained images of sectioned femur tissues of a 150 mg/mL LASCol solid implanted population taken 28 days after implantation, in accordance with Example of the present invention.
- Fig. 23: is a graph showing results of evaluating sectioned femur tissues 28 days after implantation based on the Allen's score in the 1 mm femur defect population, the 50 mg/mL LASCol solid implanted population, the 100 mg/mL LASCol solid implanted population, and the 150 mg/mL LASCol solid implanted population in accordance with Example of the present invention.
- Fig. 24: shows a µCT image of a CaCO₃-containing 150 mg/mL LASCol solid implanted individual taken 14 days after implantation, in accordance with Example of the present invention.
- (a) of Fig. 25: shows a µCT image of a bFGF-containing 100 mg/mL LASCol solid implanted individual taken 35 days after implantation, in accordance with Example of the present invention.
- (b) of Fig. 25: shows a µCT image of a 4 mm femur defect individual taken 35 days after implantation, in accordance with Example of the present invention.

### Description of Embodiments

The following description will discuss an embodiment of the present invention. The present invention is, however, not limited to the embodiment below. The present invention is not limited to arrangements described below, but may be altered in various ways by a skilled person within the scope of the claims. The present invention also encompasses, in its technical scope, any embodiment and any working example derived by appropriately combining technical means disclosed in differing embodiments. Moreover, all literatures described in this specification are incorporated herein as reference literatures. Any numerical range "A to B" expressed in the present specification intends to mean "not less than A and not more than B".

### [1. Conception by inventors]

Collagen, which is a protein constituting a connective tissue between cells or a bone tissue of an animal, or gelatin, which is a thermally denatured material of collagen, can be used as a biomaterial to be implanted in a living body (e.g., as a biomaterial to complement a defective or damaged biological tissue) or as an ex vivo material (e.g., an ex vivo material for cell culture).

Collagen and atelocollagen are poorly soluble in water. Therefore, in a case where a biomaterial or an ex vivo material is prepared using an aqueous solution in which collagen or atelocollagen is dissolved, only a solution-state substance (gel) with a low concentration or a solid-state substance (sponge) with a low density can be prepared. The solution-state substance has a maximum concentration of approximately 20 mg/mL, and the solid-state substance has a maximum density of approximately 26 mg/cm³. Such a low-concentration or low-density collagen preparation has a disadvantage of extremely low mechanical strength as a biomaterial or an ex vivo material. In addition, the density of collagen present in living organisms is 200 mg/cm³ or more, and conventional collagen preparations can reproduce only approximately 1/10 of that density.

In order to overcome the disadvantage, a method may be employed which increases the mechanical strength by disorderly crosslinking collagen with collagen by heat, light, a chemical substance, or the like. This method increases the mechanical strength as a biomaterial or an ex vivo material but has a risk of increasing antigenicity (immunogenicity) in a living body. Further, in this method, even if a sponge-like solid having a large porosity is crosslinked, it is difficult to reduce voids in the sponge-like solid. Thus, although an elastic solid can be obtained by this method, it is not possible to obtain hard solids having a high density, such as wood blocks and metals. Therefore, conventionally, there has been no idea per se to use a dense freeze-dried product of collagen as a biomaterial or an ex vivo material.

Gelatin is more hydrophilic and more soluble in water, as compared with collagen. Therefore, if a biomaterial or an ex vivo material is prepared using an aqueous solution in which gelatin is dissolved, a density of the gelatin preparation can be increased up to 800 mg/cm³. Thus, the gelatin preparation can be prepared as a solid-state substance having a high density. However, gelatin has great solubility in water. Therefore, in a case where the gelatin preparation is injected into a living body, the gelatin preparation is immediately dissolved. In addition, gelatin is rapidly degraded by endogenous peptidases. Therefore, in vivo retention of the gelatin preparation is extremely low. It may also be conceivable to crosslink gelatin with gelatin as with collagen to control biodegradability of gelatin preparations. However, such a method has a risk of increasing antigenicity (immunogenicity) in a living body.

In other words, in collagen preparations and gelatin preparations, it is technically difficult to collect a large amount of the same crosslinked products by carrying out a highly reproducible crosslinking treatment, and further, there are many problems concerning in vivo safety of such crosslinked products. In the conventional technique, only a mixture of crosslinked products having different crosslinking numbers and crosslinking positions and having various molecular sizes is obtained. In the conventional technique, it is impossible to prepare a mixture of crosslinked products having the same crosslinking number and crosslinking position and having the same porosity.

In order to utilize collagen as a biomaterial or an ex vivo material, the present inventors have attempted to develop a new technique for producing a collagen preparation having a high concentration or a high density without crosslinking. If a collagen preparation having a high concentration or a high density comparable to the collagen concentration or density in a living body is realized, such a collagen preparation is expected to have high mechanical characteristics which are not conventionally seen, and is expected to be utilized not only in vivo but also in vitro.

The collagen preparation having a high concentration or a high density may be utilized in vivo as a biomaterial that allows bone regeneration in a bone defect patient. Specifically, if a bone is defective, complete self-renewal is difficult. Therefore, bone regeneration therapy using an autologous bone or a bone derived from a conspecific organism has been conventionally carried out. For transplantation of an autologous bone, it is necessary to extract a portion of one's own bone, and there is a limit to an amount of bone that can be extracted. Meanwhile, for transplantation of a bone derived from a conspecific organism, a bone of another person is used. Therefore, a risk of infection is high. It is also conceivable that bone regeneration therapy is carried out using, as an artificial bone filler, a bone filler composed of calcium phosphate such as hydroxyapatite or β-TCP as a base material. A mixture of calcium phosphate and collagen or gelatin has been commercialized as a bone filler. In order to increase affinity for cells in vivo, calcium phosphate and collagen or gelatin can be mixed to form a bone filler. However, the bone filler does not have sufficient mechanical strength as a bone filler. Therefore, in the conventional technique, in order to improve the mechanical strength, a ratio of calcium phosphate is higher than that of collagen or gelatin. However, the bone filler itself described above does not have an ability to create a new bone, and a rate of bone formation is significantly inferior to that of an autologous bone. In recent years, bone regeneration therapy has been carried out using stem cells and scaffolds formed of bone filler. However, there are many problems in this technique, such as the need for a cell culture facility and a high cost. Under the circumstances, the present inventors have attempted to develop a new technique for bone regeneration therapy.

As a utilization method of collagen preparation having a high concentration or a high density in vivo and in vitro, the collagen preparation can be expected to be utilized as a three-dimensional scaffold for cell culture. In a case where a conventional commercially available collagen solution is used as a scaffold, the collagen solution which has a low concentration of 3 mg/mL is applied on a substrate to form a scaffold, or the gelled collagen solution is used as a scaffold. In this case, the concentration of collagen in the scaffold is greatly different from the in vivo environment, and it is therefore difficult to know functions and behavior of original cells. For example, in a case where primary cells taken from a tissue or the like are cultured on a scaffold having a low concentration of collagen, the cell cycle progresses faster, and cell growth is easily started. Although this case is advantageous to increase the number of primary cells, such extreme cell growth does not occur in a living body. Moreover, in such an environment, primary cells are generally prone to dedifferentiation. Once the primary cells are dedifferentiated, the function of the primary cells is lost, and this makes it impossible to investigate the function of the primary cells. In other words, there are many problems in the technique of confirming the original function of cells using conventional commercially available collagen solutions. Therefore, it is urgently necessary to develop a new scaffold imitating the in vivo environment in the field of regenerative medical techniques, the field of cell biology, the field of developmental biology, and the like.

For example, Patent Literature 1 discloses a composite type of bone filling material composed of a combination of collagen and a calcium phosphate compound. Patent Literature 2 discloses a biomaterial containing collagen, calcium phosphate and sugar as main components. Non-patent Literature 1 discloses a biomaterial composed of collagen crosslinked by glutaraldehyde.

In addition, Patent Literature 3 discloses a degradation product of collagen or atelocollagen, a method for producing the degradation product, and use of the degradation product. The degradation product has spheroid-forming activity. Meanwhile, Patent Literature 4 discloses a differentiation-inducing composition containing a degradation product of collagen or atelocollagen. The differentiation-inducing composition has spheroid-forming activity, bone differentiation inducing ability, and the like.

Sites in a living body where a biomaterial is to be implanted often have complex shapes. In order for the biomaterial to function well in the living body, it is important to adapt a shape of the biomaterial to a shape of a site at which the biomaterial is to be implanted. However, the conventional biomaterial and ex vivo material have a problem that the biomaterial and the ex vivo material do not have a high density and high strength (hardness) (in other words, the conventional biomaterial and ex vivo material are soft) and therefore cannot be processed into an intended shape. That is, the conventional biomaterial and ex vivo material are sponge-like solids having a high porosity, and high strength (hardness) cannot be given to such biomaterial and ex vivo material. This problem is common to all of known sponge-like solids prepared from protein components and cannot be solved. Nobody could imagine making uniform solids having few voids (like metal) with only a protein. If a solid having a high density and high strength (hardness) can be prepared from a single collagen, it will be possible to open up an entirely new application for the solid. If a solid having a high density and high strength (hardness) can be prepared from a single collagen, it is possible to prepare a solid having an intended shape (e.g., a cubic shape, a columnar shape, a disk shape, a straight tube shape, a curved tube shape, a screw shape, a male screw shape, a female screw shape, a film shape, and the like) by using an appropriate template. Preparing such a shaped product using collagen originally present in a living body is a breakthrough and the utility value of such a shaped product is immeasurable.

The degradation product disclosed in Patent Literature 3 is in the form of solution. Patent Literature 3 does not disclose a concept of concentrating the degradation product into a highly dense solid. Of course, Patent Literature 3 does not disclose a tangent modulus of the solid. Further, the differentiation-inducing composition disclosed in Patent Literature 4 is also in the form of solution. Patent Literature 4 does not disclose a concept of concentrating the degradation product into a highly dense solid. Of course, Patent Literature 4 does not disclose a tangent modulus of the solid.

Indeed, a solid having a high density is not known so far which is prepared by freeze-drying an aqueous solution containing a single undenatured protein at a concentration of 50 mg/mL or more. If such a solid can be prepared, such a solid can be utilized as an entirely new biomaterial or ex vivo material.

For example, both of the degradation product disclosed in Patent Literature 3 and the differentiation-inducing composition disclosed in Patent Literature 4 are in the form of solution or in a low density state. The degradation product and differentiation-inducing composition which are implanted into living bodies easily diffuse and are lost from the implanted sites. As such, there has been room for further improvement in regeneration of a living tissue (e.g., bone) at a target site.

In addition, a bone (e.g., femur) is a biological tissue to which a large load is to be applied and, when a biomaterial is implanted into a bone or the like, it is demanded that the biomaterial itself has high strength. However, the conventional biomaterial has a problem that the biomaterial does not have high strength (in other words, the conventional biomaterial is soft) and therefore can be implanted into only limited biological tissues.

Furthermore, conventionally, collagen has been widely used as an ex vivo material, i.e., a scaffold for cell culture. However, in the conventional technique, a collagen-containing aqueous solution at a low concentration of approximately 3 mg/mL is applied to a culture dish, and cells are cultured on the culture dish. Alternatively, a collagen-containing aqueous solution having a low concentration of approximately 3 mg/mL is gelled on a culture dish, and then cells are cultured on the culture dish. However, the concentration of collagen in vivo is not the low concentration of approximately 3 mg/mL but is a high concentration. Therefore, the conventional technique has a problem that cells cannot be cultured in vitro in a condition similar to an in vivo condition. Further, there is a problem that a shaped product having a shape (e.g., a cube) suitable for culture cannot be prepared.

In order to achieve high strength in a conventional biomaterial or ex vivo material, it is necessary to employ an auxiliary material different from collagen (e.g., a crosslinking agent or a synthetic polymer). However, the auxiliary material has a problem that the auxiliary material increases a cost of biomaterial and/or increases immunogenicity, inflammation, retention, and the like of the biomaterial in a living body, thereby reducing safety. Further, even if a large amount of auxiliary material is added to the biomaterial or ex vivo material to increase the strength of the biomaterial or ex vivo material, the density of collagen itself contained in the biomaterial or ex vivo material cannot be increased. That is, there is a problem that the density of collagen present in a living body cannot be reproduced using conventional collagen. In other words, in the conventional technique, although it is possible to prepare a sponge-like solid which is reinforced in strength and has a high porosity from a collagen preparation or a gelatin preparation, it has been impossible to prepare a uniform collagen solid or gelatin solid having little porosity like metals. It is conceivable to prepare a highly dense solid by compressing the sponge-like solid. However, when the compressive force is released, the solid expands back to the original sponge-like solid. In order to obtain a highly dense solid, it is also conceivable to crosslink the sponge-like solid in a compressed state. However, such a crosslinked solid is an artifact that does not exist in nature.

In addition, conventional biomaterials or ex vivo materials have a problem that it is difficult to incorporate an optional substance component into the biomaterial or ex vivo material because, for example, solubility of the raw material is low or strength of the biomaterial or ex vivo material is low.

An object of the present invention is to provide a collagen solid having a high density and high strength (in other words, a shaped product which is close to an in vivo environment and has a low porosity).

### [2. Method for producing collagen solid]

The method in accordance with an embodiment of the present invention for producing a collagen solid includes (i) a degradation step of degrading collagen or atelocollagen with a cysteine protease (specifically, partially cutting both ends of collagen or atelocollagen with the cysteine protease) and (ii) a removal step of removing a solvent from a collagen degradation product or an atelocollagen degradation product which has been obtained in the degradation step. The following description will discuss those steps.

### [2-1. Degradation step]

In the degradation step, collagen or atelocollagen is degraded with a cysteine protease.

The collagen is not limited to any particular one, and may be any well-known collagen. Examples of the collagen include collagens of (i) mammals (for example, a cow, a pig, a rabbit, a human, a rat, and a mouse), (ii) birds (for example, a chicken), or (iii) fishes (for example, a shark, a carp, an eel, a tuna [for example, a yellowfin tuna], a tilapia, a sea bream, and a salmon).

Further specifically, examples of the collagen include (i) collagen derived from, for example, a dermis, a tendon, a bone, or a fascia of any of mammals or birds and (ii) collagen derived from, for example, a skin or a scale of any of fishes.

Examples of the atelocollagen include atelocollagen which is produced by treating collagen of any of mammals, birds, or fishes with a protease (for example, pepsin) and in which a telopeptide(s) has been partially removed from the amino terminus and/or carboxyl terminus of the collagen molecules.

A preferable option among the above examples is collagen or atelocollagen of a chicken, a pig, a human, or a rat. A further preferable option among the above examples is collagen or atelocollagen of a pig or a human.

Collagen or atelocollagen of a fish can be prepared safely in a large amount, and it is possible to provide a collagen solid that is safer with respect to humans.

In a case where collagen or atelocollagen of a fish is used, (i) a preferable option is collagen or atelocollagen of a shark, a carp, an eel, a tuna (for example, a yellowfin tuna), a tilapia, a sea bream, or a salmon, and (ii) a further preferable option is collagen or atelocollagen of a tuna, a tilapia, a sea bream, or a salmon.

The collagen may be prepared by a well-known method. For example, collagen-rich tissue of a mammal, a bird, or a fish is put into an acid solution with a pH of approximately 2 to 4 for elution of collagen. Further, a protease such as pepsin is added to the eluate for partial removal of a telopeptide(s) at the amino terminus and/or carboxyl terminus of the collagen molecules. Then, a salt such as sodium chloride is added to the eluate to precipitate atelocollagen.

In a case where atelocollagen is used, the atelocollagen has a heat denaturation temperature of preferably not lower than 15°C, more preferably not lower than 20°C. In a case where, for example, atelocollagen of a fish is used, the atelocollagen is preferably derived from a tuna (for example, a yellowfin tuna), a tilapia, a carp, or the like because such atelocollagen has a heat denaturation temperature of not lower than 25°C. The above feature allows for production of a collagen solid that is excellent in stability in storage and in use.

The cysteine protease is preferably (i) a cysteine protease that contains a larger amount of acidic amino acids than that of basic amino acids or (ii) a cysteine protease that is active at a hydrogen ion concentration in an acidic region.

Examples of such a cysteine protease include cathepsin B [EC 3.4.22.1], papain [EC 3.4.22.2], ficin [EC 3.4.22.3], actinidain [EC 3.4.22.14], cathepsin L [EC 3.4.22.15], cathepsin H [EC 3.4.22.16], cathepsin S [EC 3.4.22.27], bromelain [EC 3.4.22.32], cathepsin K [EC 3.4.22.38], alloline, and calcium dependent protease.

Among those, it is preferable to use papain, ficin, actinidain, cathepsin K, alloline, or bromelain, and it is further preferable to use papain, ficin, actinidain, or cathepsin K.

The enzyme can be prepared by a publicly known method. Examples of such a method include (i) a method of preparing an enzyme by chemical synthesis; (ii) a method of extracting an enzyme from a bacterium, a fungus, or a cell or tissue of any of various animals and plants; and (iii) a method of preparing an enzyme by a genetic engineering means. The enzyme can alternatively be a commercially available enzyme as well.

In a case where collagen or atelocollagen is degraded with use of an enzyme (specifically, a cysteine protease), the degradation can be carried out by, for example, any of the methods (i) through (iii) below. The methods (i) through (iii) below are, however, mere examples, and the present invention is not limited to the methods (i) through (iii).

The methods (i) and (ii) below are each an example method for cleaving a chemical bond at a particular position in the amino acid sequence in (1) or (2) described later, and the method (iii) below is an example method for cleaving a chemical bond at a particular position in the amino acid sequence in (3) described later.
(i) Method of causing collagen or atelocollagen to be in contact with an enzyme in the presence of a salt having a high concentration.
(ii) Method of causing collagen or atelocollagen to be in contact with an enzyme having been in contact with a salt having a high concentration.
(iii) Method of causing collagen or atelocollagen to be in contact with an enzyme in the presence of a salt having a low concentration.

A specific example of the method (i) above is a method of causing collagen or atelocollagen to be in contact with an enzyme in an aqueous solution containing a salt at a high concentration.

A specific example of the method (ii) above is a method of causing an enzyme to be in contact in advance with an aqueous solution containing a salt at a high concentration and then causing collagen or atelocollagen to be in contact with that enzyme.

A specific example of the method (iii) above is a method of causing collagen or atelocollagen to be in contact with an enzyme in an aqueous solution containing a salt at a low concentration.

The aqueous solution is not particularly limited in terms of specific arrangements. The aqueous solution can, for example, contain water as a solvent.

The salt is not particularly limited in terms of specific arrangements, but is preferably a chloride. The chloride is not limited to any particular one. Examples of the chloride include NaCl, KCI, LiCI, and MgCl₂.

The salt contained in the aqueous solution at a high concentration may have any concentration. A higher concentration is, however, more preferable. The concentration is, for example, preferably not less than 200 mM, more preferably not less than 500 mM, even more preferably not less than 1000 mM, even more preferably not less than 1500 mM, most preferably not less than 2000 mM.

The concentration of the salt contained in the aqueous solution at a high concentration may have any upper limit. The upper limit may be 2500 mM, for example. A salt concentration of higher than 2500 mM will salt out a large amount of protein, with the result that the enzymatic degradation of collagen or atelocollagen tends to have a decreased efficiency. A salt concentration of not more than 2500 mM allows for a higher efficiency of enzymatic degradation of collagen or atelocollagen.

It follows that the concentration of the salt contained in the aqueous solution at a high concentration is preferably within a range of not less than 200 mM and not more than 2500 mM, more preferably within a range of not less than 500 mM and not more than 2500 mM, even more preferably within a range of not less than 1000 mM and not more than 2500 mM, even more preferably within a range of not less than 1500 mM and not more than 2500 mM, most preferably within a range of not less than 2000 mM and not more than 2500 mM.

A higher concentration of the salt contained in the aqueous solution at a high concentration can increase the specificity at the position of the enzymatic cleavage of a chemical bond in collagen or atelocollagen.

The salt contained in the aqueous solution at a low concentration may have any concentration. A lower concentration is, however, more preferable. The concentration is, for example, preferably lower than 200 mM, more preferably not more than 150 mM, even more preferably not more than 100 mM, even more preferably not more than 50 mM, most preferably substantially 0 mM.

Collagen or atelocollagen may be dissolved in the aqueous solution (for example, water) in any amount. For example, 1 part by weight of collagen or atelocollagen is preferably dissolved in 100 parts by weight to 10000 parts by weight of the aqueous solution. Further, 1 part by weight of collagen or atelocollagen is preferably dissolved in 100 parts by weight to 1000 parts by weight of the aqueous solution.

With the above feature, in a case where the enzyme has been added to the aqueous solution, the enzyme comes into contact efficiently with the collagen or atelocollagen. This in turn allows the collagen or atelocollagen to be degraded efficiently with use of the enzyme.

The enzyme may be added to the aqueous solution in any amount. For example, 1 part by weight to 100 parts by weight of the enzyme is preferably added to 1000 parts by weight of the collagen or atelocollagen. With the above feature, the aqueous solution has a high enzyme concentration. This allows the collagen or atelocollagen to be degraded efficiently with use of the enzyme. Further, 1 part by weight to 10 parts by weight of the enzyme is preferably added to 100 parts by weight of the collagen or atelocollagen.

Other conditions (for example, the pH and temperature of the aqueous solution, and the contact period) under which the collagen or atelocollagen is caused to be in contact with the enzyme in the aqueous solution are not particularly limited, and may be set as appropriate. Such other conditions are, however, preferably within the ranges below.
(1) The aqueous solution has a pH of preferably 2.0 to 7.0, further preferably 2.5 to 6.5. The aqueous solution can contain a well-known buffer to have a pH kept within the above range. The aqueous solution having a pH within the above range allows collagen or atelocollagen to be dissolved therein uniformly, and consequently allows an enzymatic reaction to occur efficiently.
(2) The temperature of the aqueous solution is not limited to any particular value, and may be selected in view of the enzyme used. The temperature is, for example, preferably within a range of 15°C to 40°C, more preferably within a range of 20°C to 35°C.
(3) The contact period is not limited to any particular length, and may be selected in view of the amount of the enzyme and/or the amount of the collagen or atelocollagen. The contact period is, for example, preferably within a range of 1 hour to 60 days, more preferably within a range of 1 day to 7 days, further preferably within a range of 3 days to 7 days.

A method for the present embodiment may include, as necessary, at least one step selected from the group consisting of a step of readjusting the pH, a step of inactivating the enzyme, and a step of removing contaminants, after the collagen or atelocollagen is caused to be in contact with the enzyme in the aqueous solution.

The step of removing contaminants can be carried out by a typical method for separating a substance. The step of removing contaminants can be carried out by, for example, dialysis, salting-out, gel filtration chromatography, isoelectric precipitation, ion exchange chromatography, or hydrophobic interaction chromatography.

The degradation step can be carried out by degrading the collagen or atelocollagen with use of the enzyme as described above. The collagen or atelocollagen to be degraded may be contained in biological tissue. In other words, the degradation step can be carried out by causing such biological tissue to be in contact with the enzyme.

The biological tissue is not limited to any particular tissue, and can be, for example, a dermis, a tendon, a bone, or a fascia of a mammal or a bird, or a skin or a scale of a fish.

The biological tissue is preferably a dermis, a tendon, or a bone from the viewpoint of maintaining high physiological activity and the ability to produce a collagen degradation product or an atelocollagen degradation product in a large amount.

In a case where the biological tissue is a dermis, a tendon, or a bone, the dermis, the tendon, or the bone is preferably caused to be in contact with the enzyme in an acidic condition. The acidic condition is, for example, preferably a pH of 2.5 to 6.5, further preferably a pH of 2.5 to 5.0, even further preferably a pH of 2.5 to 4.0, most preferably a pH of 2.5 to 3.5.

More specifically, in the degradation step, it is preferable to cause a dermis, a tendon, or a bone to be in contact with the cysteine protease so that collagen contained in the dermis, the tendon, or the bone is caused to be in contact with the cysteine protease. In the degradation step, it is preferable to cause the dermis, the tendon, or the bone to be in contact with the cysteine protease in the presence of a salt at a concentration of not less than 200 mM. In the degradation step, it is preferable to cause the dermis, the tendon, or the bone to be in contact with a cysteine protease having been in contact with a salt at a concentration of not less than 200 mM. In the degradation step, it is preferable to cause the dermis, the tendon, or the bone to be in contact with the cysteine protease in the presence of a salt at a concentration of lower than 200 mM.

### [2-2. Removal step]

The removal step is a step of removing the solvent from the collagen degradation product or the atelocollagen degradation product obtained in the degradation step. In the removal step, not only the solvent but also impurities such as unnecessary low molecular weight compounds may be removed. The removal step can be carried out, for example, by dialysis, ultrafiltration, freeze drying, air drying, evaporator, spray drying, or a combination of these.

The above dialysis or ultrafiltration can remove impurities such as unnecessary low molecular weight compounds other than a solvent (e.g., water). Dialysis or ultrafiltration may be repeated until the amount of unnecessary low molecular weight compounds contained in the solvent becomes negligible, and dialysis and ultrafiltration may be carried out in combination. From the viewpoint of preventing the collagen solid from denaturing, the removal step is preferably carried out at a low temperature. Note that, since the methods such as dialysis and ultrafiltration above are well known, descriptions of such methods are omitted here.

The freeze drying, air drying, evaporator or spray drying can remove a solvent such as water. From the viewpoint of preventing the collagen solid from denaturing, the removal step is preferably carried out at a low temperature. In a freezing step in pretreatment of the freeze drying, the collagen solid may be frozen using an ultracold freezer at -80°C. Alternatively, a program freezer may be used to cool and freeze the collagen solid to a final temperature of -80°C. Alternatively, the collagen solid may be frozen using an ultracold freezer at -80°C after pre-freezing the collagen solid using a program freezer. Since the methods such as freeze drying, air drying, evaporator, and spray drying above are well known, descriptions of such methods are omitted here.

In the removal step (e.g., the freeze drying step or the like), it is preferable to fill a template having an intended shape with the collagen degradation product or the atelocollagen degradation product and then remove the solvent from the collagen degradation product or the atelocollagen degradation product. With this process, the collagen solid having an intended shape can be easily obtained. In the spray drying step, it is preferable to spray the collagen degradation product or the atelocollagen degradation product in the form of mist, and then remove the solvent from the collagen degradation product or the atelocollagen degradation product. With this process, the collagen solid in an intended form of powder can be easily obtained.

In the removal step, it is possible that an optional substance is added to the collagen degradation product or the atelocollagen degradation product which has been obtained in the degradation step to obtain a mixture, and then the solvent is removed from the mixture. Specifically, in the removal step, it is possible that an optional substance dissolved in a certain solvent is added to the collagen degradation product or the atelocollagen degradation product which has been obtained in the degradation step to obtain a mixture, and then the solvent is removed from the mixture. In the removal step, it is possible that a collagen solid which has been obtained in the removal step is caused to adsorb an optional substance. Specifically, in the removal step, it is possible that a collagen solid which has been obtained in the removal step is caused to absorb an optional substance dissolved in a certain solvent. More specifically, in the removal step, it is possible that a collagen solid which has been obtained in the removal step is caused to absorb an optional substance dissolved in a certain solvent, and then the solvent in which the optional substance is dissolved is removed from the collagen solid. Further specifically, in the removal step, it is possible that (i) a collagen solid is obtained by removing the solvent from the collagen degradation product or the atelocollagen degradation product which has been obtained in the degradation step, (ii) the collagen solid is immersed in a solvent containing an optional substance (in other words, an optional substance dissolved in a certain solvent), and (iii) the solvent is removed from the collagen solid. According to the process, it is possible to produce the collagen solid containing the optional substance.

### [2-3. Other step]

The method for producing the collagen solid in accordance with an embodiment of the present invention can include, after the above-described removal step, a shaping step of further applying a shaping process (e.g., a cutting process, a polishing process, a through hole forming process, and the like) to the collagen solid obtained in the removal step. With this feature, the collagen solid having an intended shape can be easily obtained. The shaping step may be carried out according to a well-known method.

In the shaping step, an appropriate template having projections and depressions corresponding to a shape of a shaped product can be used. The shape of the shaped product prepared with use of the template includes, for example, a cubic shape, a columnar shape, a disk shape, a straight tube shape, a curved tube shape, a screw shape, a male screw shape, a female screw shape, a film shape, a conical shape, an arrowhead shape, a hexahedral shape, a polyhedral shape, a polygonal column shape, a bellows shape, and a complex shape in which two or more of these shapes are connected to each other.

### [3. Collagen solid]

The collagen solid in accordance with an embodiment of the present invention can be prepared by the production method described in the section of [2. Method for producing collagen solid] above. The collagen solid in accordance with an embodiment of the present invention includes a collagen-cysteine protease degradation product or an atelocollagen-cysteine protease degradation product. The following description will discuss the individual features. Note that, in regard to the features described above in the section of [2. Method for producing collagen solid], descriptions of such features will be omitted below.

### [3-1. Properties of collagen solid]

A density of the collagen solid in accordance with an embodiment of the present invention is preferably approximately 50 mg/cm³ or more, more preferably approximately 50 mg/cm³ to approximately 400 mg/cm³, more preferably approximately 50 mg/cm³ to approximately 350 mg/cm³, more preferably approximately 80 mg/cm³ to approximately 350 mg/cm³, more preferably approximately 80 mg/cm³ to approximately 300 mg/cm³, more preferably approximately 100 mg/cm³ to approximately 300 mg/cm³, more preferably approximately 120 mg/cm³ to approximately 300 mg/cm³, more preferably approximately 120 mg/cm³ to approximately 280 mg/cm³, more preferably approximately 140 mg/cm³ to approximately 280 mg/cm³, more preferably approximately 140 mg/cm³ to approximately 260 mg/cm³, more preferably approximately 140 mg/cm³ to approximately 240 mg/cm³, most preferably approximately 140 mg/cm³ to approximately 220 mg/cm³.

A method of measuring the density of the collagen solid is not particularly limited, and for example, the density can be measured by a method described in Examples described later.

A tangent modulus of the collagen solid in accordance with an embodiment of the present invention is preferably approximately 90 kPa or more, more preferably approximately 90 kPa to approximately 40000 kPa, more preferably approximately 90 kPa to approximately 35000 kPa, more preferably approximately 150 kPa to approximately 35000 kPa, more preferably approximately 200 kPa to approximately 35000 kPa, more preferably approximately 200 kPa to approximately 30000 kPa, more preferably approximately 200 kPa to approximately 25000 kPa, more preferably approximately 250 kPa to approximately 25000 kPa, more preferably approximately 300 kPa to approximately 25000 kPa, more preferably approximately 300 kPa to approximately 20000 kPa, more preferably approximately 300 kPa to approximately 15000 kPa, most preferably approximately 300 kPa to approximately 10000 kPa.

A method of measuring the tangent modulus of the collagen solid is not particularly limited, and for example, the tangent modulus can be measured by a method described in Examples described later.

The collagen solid in accordance with an embodiment of the present invention can have the density described above, can have the tangent modulus described above, and can have both the density and the tangent modulus described above.

An amount of each of the collagen-cysteine protease degradation product and the atelocollagen-cysteine protease degradation product contained in the collagen solid in accordance with an embodiment of the present invention is not particularly limited. However, a larger amount of these degradation products is preferable because the strength of the collagen solid is improved. For example, a total amount of each of the degradation products in the collagen solid in accordance with an embodiment of the present invention can be preferably 0.1% by weight to 100% by weight, more preferably 50% by weight to 100% by weight, more preferably 90% by weight to 100% by weight, most preferably 100% by weight.

To the collagen solid in accordance with an embodiment of the present invention, components other than the collagen-cysteine protease degradation product and the atelocollagen-cysteine protease degradation product can be added. These components are not particularly limited. Examples of these components include elements (e.g., calcium, magnesium, potassium, sodium, chloride, zinc, iron, and copper, or ions thereof), inorganic acids (phosphoric acid, acetic acid, and carbonic acid, or ions thereof), organic acids (pyruvic acid, acetyl-CoA, citric acid, oxalacetic acid, succinic acid, and fumaric acid, or ions thereof), low molecular weight compounds (e.g., CaCO₃) nucleic acids (DNA, RNA, plasmids), nucleosides, nucleotides, ATP, GTP, NADH, FADH₂, siRNA, miRNA, lipids, amino acids, proteins, cytokines, growth factors (e.g., FGF, bFGF, VEGF, BMP, TGF-β, PDGF, HGF, and IGF), monosaccharides (glucose, fucose, glucosamine), polysaccharides (hyaluronic acid, trehalose, amylose, pectin, cellulose, glycogen, starch, and chitin), chemically synthesized drugs, natural drugs, enzymes, hormones (testosterone, dihydrotestosterone, estrone, estradiol, progesterone, luteinizing hormone, follicle-stimulating hormone, thyroid hormone), antibiotics, anticancer drugs, proteoglycans, antibodies, exosomes, and cytoclastic components, mixtures thereof, and the like.

In a case where the collagen solid in accordance with an embodiment of the present invention includes components other than the collagen-cysteine protease degradation product and the atelocollagen-cysteine protease degradation product, the collagen solid can be obtained as follows: (i) the collagen-cysteine protease degradation product and/or the atelocollagen-cysteine protease degradation product, a solvent, and components other than the collagen-cysteine protease degradation product and the atelocollagen-cysteine protease degradation product are mixed and then the solvent is evaporated to obtain a collagen solid which contains other components or (ii) the collagen-cysteine protease degradation product and/or the atelocollagen-cysteine protease degradation product and a solvent are mixed and dried to obtain a collagen solid, then the collagen solid thus obtained is caused to absorb components other than the collagen-cysteine protease degradation product and the atelocollagen-cysteine protease degradation product, and then unnecessary solvent and the like are evaporated to obtain a collagen solid containing other components. Alternatively, the collagen-cysteine protease degradation product or the atelocollagen-cysteine protease degradation product is mixed with a plurality of components other than the collagen-cysteine protease degradation product or the atelocollagen-cysteine protease degradation product, and then unnecessary solvent and the like are evaporated to obtain a collagen solid containing the plurality of components.

In the collagen solid in accordance with an embodiment of the present invention, components other than the collagen-cysteine protease degradation product and the atelocollagen-cysteine protease degradation product can be contained in a total amount of 0% by weight to 99.9% by weight, 0% by weight to 50% by weight, 0% by weight to 10% by weight, or 0% by weight.

The collagen solid in accordance with an embodiment of the present invention can have been processed to have an intended shape. Examples of the shape include a disk shape, a tube shape, a columnar shape, a conical shape, an arrowhead shape, a hexahedral shape, a polyhedral shape, a polygonal column shape, a bellows shape, a screw shape, a male screw shape, a female screw shape and a complex shape in which two or more of these shapes are connected to each other. Of course, however, the present invention is not limited to these shapes.

### [2-2. Collagen-cysteine protease degradation product and atelocollagen-cysteine protease degradation product contained in collagen solid]

Each of the collagen-cysteine protease degradation product and the atelocollagen-cysteine protease degradation product can contain at least a part of a triple helical domain of collagen. The degradation product may, in other words, contain the entire triple helical domain of collagen or a portion of the triple helical domain.

More specifically, each of the collagen-cysteine protease degradation product and the atelocollagen-cysteine protease degradation product can be a degradation product of collagen or atelocollagen which degradation product results from:
cleavage of a chemical bond between X₁ and X₂, between X₂ and G, between G and X₃, between X₄ and G, or between X₆ and G in an amino acid sequence in (1) below within the triple helical domain of collagen or atelocollagen;
cleavage of a chemical bond between X₁ and X₂, between X₂ and G, between G and X₃, between X₄ and G, between X₆ and G, between G and X₇, or between X₁₄ and G in an amino acid sequence in (2) below within the triple helical domain of collagen or atelocollagen; or
cleavage of a chemical bond between Y₁ and Y₂ in an amino acid sequence in (3) below at an amino terminus of the triple helical domain of collagen or atelocollagen.
   (1) -G-X₁-X₂-G-X₃-X₄-G-X₅-X₆-G- (SEQ ID NO: 1),
   (2) -G-X₁-X₂-G-X₃-X₄-G-X₅-X₆-G-X₇-X₈-G-X₉-X₁₀-G-X₁₁-X₁₂-G-X₁₃-X₁₄-G- (SEQ ID NO: 2),
   (3) -Y₁-Y₂-Y₃-G-Y₄-Y₅-G-Y₆-Y₇-G-Y₈-Y₉-G- (SEQ ID NO: 3),
where G represents glycine, and X₁ to X₁₄ and Y₁ to Y₉ each represent any amino acid.

The term "triple helical domain" as used in the present specification intends to mean a domain that (i) contains not fewer than 3, preferably not fewer than 80, more preferably not fewer than 100, more preferably not fewer than 200, more preferably not fewer than 300, units of amino acid sequences in tandem each of which units is represented as "Gly-X-Y" (where X and Y each represent an amino acid) and that (ii) contributes to formation of a helical structure.

The cleavage of a chemical bond within the triple helical domain may occur in any of a plurality of kinds of polypeptide chains included in the collagen. The cleavage of a chemical bond may occur in, for example, any of the following polypeptide chains: the α1 chain, the α2 chain, and the α3 chain. The cleavage of a chemical bond occurs preferably in at least one of the α1 chain and the α2 chain among the above polypeptide chains. The cleavage of a chemical bond occurs further preferably in the α1 chain among the above polypeptide chains.

Each of the collagen-cysteine protease degradation product and the atelocollagen-cysteine protease degradation product may contain three polypeptide chains in a helical structure. Each of the collagen-cysteine protease degradation product and the atelocollagen-cysteine protease degradation product may alternatively contain three polypeptide chains that are not in a helical structure entirely or partially. Whether the three polypeptide chains are in a helical structure can be determined by a publicly known method (for example, by observing a circular dichroism spectrum of the degradation product).

Each of the collagen-cysteine protease degradation product and the atelocollagen-cysteine protease degradation product basically contains three polypeptide chains. The cleavage of a chemical bond may occur in one, two, or all of the three polypeptide chains.

In a case where the degradation product of collagen or atelocollagen contains three polypeptide chains in a helical structure, a plurality of helical structures may form a meshwork assembly or filamentous assembly. The term "meshwork" as used in the present specification intends to describe a structure of molecules connected to one another through, for example, hydrogen bonding, electrostatic interaction, or van der Waals bonding to form a three-dimensional mesh and openings therein. The term "filamentous" as used in the present specification intends to describe a substantially linear structure of molecules connected to one another through, for example, hydrogen bonding, electrostatic interaction, or van der Waals bonding. The term "assembly" as used in the present specification intends to mean a structural unit of two or more molecules of an identical kind that bond to one another not through covalent bonding but through interaction with one another. Whether a meshwork or filamentous assembly is present can be determined by observing the degradation product with an electron microscope.

The amino acid sequence in (1) or (2) above may be at any position within the triple helical domain. The amino acid sequence in (1) or (2) above may be, for example, at a position away from the two terminuses of the triple helical domain, but is preferably at the amino terminus of the triple helical domain. Stated differently, that "G" in the amino acid sequence in (1) or (2) above which is closest to the amino terminus preferably corresponds to that "G" within the triple helical domain which is closest to the amino terminus.

Each of the amino acid sequences in (1), (2) and (3) may be connected, at the amino terminus of each of the amino acid sequences in (1), (2) and (3), to not fewer than 1, not fewer than 5, not fewer than 10, not fewer than 50, not fewer than 100, not fewer than 150, not fewer than 200, not fewer than 250, or not fewer than 300, units of amino acid sequences in tandem each of which units is represented as "Gly-X-Y" (where X and Y each represent an amino acid). Each of the amino acid sequences in (1), (2) and (3) may be connected, at the carboxyl terminus of each of the amino acid sequences in (1), (2) and (3), to not fewer than 1, not fewer than 5, not fewer than 10, not fewer than 50, not fewer than 100, not fewer than 150, not fewer than 200, not fewer than 250, or not fewer than 300, units of amino acid sequences in tandem each of which units is represented as "Gly-X-Y" (where X and Y each represent an amino acid).

X₁ to X₆ can each be any amino acid, and are each not limited to any particular kind. At least two of X₁ to X₆ may be amino acids of an identical kind. X₁ to X₆ may alternatively be amino acids all of which differ from one another in kind.

X₁ to X₆ may each be, for example, any of the following amino acids: glycine, alanine, valine, leucine, isoleucine, serine, threonine, tyrosine, cysteine, methionine, aspartic acid, asparagine, glutamic acid, glutamine, arginine, lysine, histidine, phenylalanine, tyrosine, tryptophan, hydroxyproline, and hydroxylysine.

Further specifically, X₁ to X₆ may be such that X₁, X₃, and X₅ are identical amino acids, while the others are different amino acids.

Further specifically, X₁ to X₆ may be such that at least one selected from the group consisting of X₁, X₃, and X₅ is proline, while the others are each any amino acid.

Further specifically, X₁ to X₆ may be such that X₁ is proline, while X₂ to X₆ are each any amino acid.

Further specifically, X₁ to X₆ may be such that X₁ and X₃ are each proline, while X₂ and X₄ to X₆ are each any amino acid.

Further specifically, X₁ to X₆ may be such that X₁, X₃, and X₅ are each proline, while X₂, X₄, and X₆ are each any amino acid.

Further specifically, X₁ to X₆ may be such that (i) X₁, X₃, and X₅ are each proline, (ii) X₂ is an amino acid containing a sulfur atom in a side chain (for example, cysteine or methionine) or an amino acid containing a hydroxyl group in a side chain (for example, hydroxyproline, hydroxylysine, or serine), and (iii) X₄ and X₆ are each any amino acid.

Further specifically, X₁ to X₆ may be such that (i) X₁, X₃, and X₅ are each proline, (ii) X₂ is an amino acid containing a sulfur atom in a side chain (for example, cysteine or methionine), (iii) X₄ is an amino acid having an aliphatic side chain (for example, glycine, alanine, valine, leucine, or isoleucine) or an amino acid containing a hydroxyl group in a side chain (for example, hydroxyproline, hydroxylysine, or serine), and (iv) X₆ is any amino acid.

Further specifically, X₁ to X₆ may be such that X₁, X₃, and X₅ are each proline, (ii) X₂ is an amino acid containing a sulfur atom in a side chain (for example, cysteine or methionine), (iii) X₄ is an amino acid having an aliphatic side chain (for example, glycine, alanine, valine, leucine, or isoleucine) or an amino acid containing a hydroxyl group in a side chain (for example, hydroxyproline, hydroxylysine, or serine), and (iv) X₆ is an amino acid containing a base in a side chain (for example, arginine, lysine, or histidine).

Further specifically, X₁ to X₆ may be such that (i) X₁, X₃, and X₅ are each proline, (ii) X₂ is methionine, (iii) X₄ is alanine or serine, and (iv) X₆ is arginine.

In the amino acid sequence in (2) above, X₁ to X₆ may be identical in arrangement to the above X₁ to X₆, respectively. The following description will discuss detailed arrangements of X₇ to X₁₄.

X₇ to X₁₄ can each be any amino acid, and are each not limited to any particular kind. At least two of X₇ to X₁₄ may be amino acids of an identical kind. X₇ to X₁₄ may alternatively be amino acids all of which differ from one another in kind.

X₇ to X₁₄ may each be, for example, any of the following amino acids: glycine, alanine, valine, leucine, isoleucine, serine, threonine, tyrosine, cysteine, methionine, aspartic acid, asparagine, glutamic acid, glutamine, arginine, lysine, histidine, phenylalanine, tyrosine, tryptophan, hydroxyproline, and hydroxylysine.

Further specifically, X₇ to X₁₄ may be such that X₈, X₉, X₁₀, X₁₂, and X₁₃ are identical amino acids, while the others are different amino acids.

Further specifically, X₇ to X₁₄ may be such that at least one selected from the group consisting of X₈, X₉, X₁₀, X₁₂, and X₁₃ is proline or hydroxyproline, while the others are each any amino acid.

Further specifically, X₇ to X₁₄ may be such that X₈ is proline or hydroxyproline, while the others are each any amino acid.

Further specifically, X₇ to X₁₄ may be such that X₈ and X₉ are each proline or hydroxyproline, while the others are each any amino acid.

Further specifically, X₇ to X₁₄ may be such that X₈, X₉, and X₁₀ are each proline or hydroxyproline, while the others are each any amino acid.

Further specifically, X₇ to X₁₄ may be such that X₈, X₉, X₁₀, and X₁₂ are each proline or hydroxyproline, while the others are each any amino acid.

Further specifically, X₇ to X₁₄ may be such that X₈, X₉, X₁₀, X₁₂, and X₁₃ are each proline or hydroxyproline, while the others are each any amino acid.

Further specifically, X₇ to X₁₄ may be such that (i) X₈, X₉, X₁₀, X₁₂, and X₁₃ are each proline or hydroxyproline, (ii) X₇ is an amino acid having an aliphatic side chain (for example, glycine, alanine, valine, leucine, or isoleucine), and (iii) the others are each any amino acid.

Further specifically, X₇ to X₁₄ may be such that (i) X₈, X₉, X₁₀, X₁₂, and X₁₃ are each proline or hydroxyproline, (ii) X₇ and X₁₁ are each an amino acid having an aliphatic side chain (for example, glycine, alanine, valine, leucine, or isoleucine), and (iii) the rest is any amino acid.

Further specifically, X₇ to X₁₄ may be such that (i) X₈, X₉, X₁₀, X₁₂, and X₁₃ are each proline or hydroxyproline, (ii) X₇ and X₁₁ are each an amino acid having an aliphatic side chain (for example, glycine, alanine, valine, leucine, or isoleucine), and (iii) X₁₄ is an amino acid having a hydrophilic and non-dissociative side chain (serine, threonine, asparagine, or glutamine).

Further specifically, X₇ to X₁₄ may be such that (i) X₈, X₉, X₁₀, X₁₂, and X₁₃ are each proline or hydroxyproline, (ii) X₇ is leucine, (iii) X₁₁ is alanine, and (iv) X₁₄ is glutamine.

The amino acid sequence in (3) above is positioned at the amino terminus of the triple helical domain. This means that (i) G between Y₃ and Y₄ indicates glycine which is within the triple helical domain and is closest to the amino terminus and that (ii) Y₁, Y₂, and Y₃ indicate amino acids which are in a plurality of kinds of polypeptide chains included in the collagen and are positioned closer to the amino terminus than the triple helical domain.

Y₁ to Y₉ can each be any amino acid, and are each not limited to any particular kind. At least two of Y₁ to Y₉ may be amino acids of an identical kind. Y₁ to Y₉ may alternatively be amino acids all of which differ from one another in kind.

Y₁ to Y₉ may each be, for example, any of the following amino acids: glycine, alanine, valine, leucine, isoleucine, serine, threonine, tyrosine, cysteine, methionine, aspartic acid, asparagine, glutamic acid, glutamine, arginine, lysine, histidine, phenylalanine, tyrosine, tryptophan, hydroxyproline, and hydroxylysine.

Further specifically, Y₁ to Y₃ may be such that Y₃ is proline, while Y₁ and Y₂ are each any amino acid.

Further specifically, Y₁ to Y₃ may be such that Y₃ is proline, while Y₁ and Y₂ are each an amino acid having an aliphatic side chain (for example, glycine, alanine, valine, leucine, or isoleucine) or an amino acid containing a hydroxyl group in a side chain (hydroxyproline, hydroxylysine, or serine).

Further specifically, Y₁ to Y₃ may be such that (i) Y₃ is proline, (ii) Y₁ is alanine or serine, and (iii) Y₂ is valine.

In any of the above cases, Y₄ to Y₉ are not particularly limited in terms of specific arrangements. Y₄ to Yg may be such that (i) Y₄ and X₁ are identical amino acids, (ii) Y₅ and X₂ are identical amino acids, (iii) Y₆ and X₃ are identical amino acids, (iv) Y₇ and X₄ are identical amino acids, (v) Y₈ and X₅ are identical amino acids, and (vi) Yg and X₆ are identical amino acids.

More specifically, X₁ and Y₄ can each be proline, X₂ and Y₅ can each be methionine, X₃ and Y₆ can each be proline or leucine, X₄ and Y₇ can each be alanine, serine, or methonine, X₅ and Y₈ can each be proline or serine, X₆ and Y₉ can each be arginine, X₇ to X₁₄ and Y₁ to Y₃ can each be any amino acid.

### [4. Biomaterial and ex vivo material]

Each of the biomaterial and the ex vivo material in accordance with an embodiment of the present invention contains the above described collagen solid.

The biomaterial can be implanted in a biological tissue (e.g., bone). The biomaterial in accordance with an embodiment of the present invention can be a biomaterial for biological tissue regeneration, or can be a biomaterial for biological tissue repairing. More specifically, the biomaterial in accordance with an embodiment of the present invention can be a biomaterial for bone regeneration (in other words, a bone regeneration material) containing the collagen solid described above. More specifically, the biomaterial in accordance with an embodiment of the present invention can be a biomaterial which contains the collagen solid described above and is used in bone regeneration for treating bone injury or a biomaterial which contains the collagen solid described above and is used in bone regeneration for treating bone defect. As also shown in Examples described later, the biomaterial in accordance with an embodiment of the present invention can effectively regenerate a bone.

The ex vivo material is not particularly limited, provided that the ex vivo material is used outside biological tissues. The ex vivo material in accordance with an embodiment of the present invention can be a substrate for cell culture or a cell culture substrate composed of a highly dense collagen solid whose shape can be processed. The ex vivo material in accordance with an embodiment of the present invention can be a cell culture substrate containing the highly dense collagen solid described above. More specifically, the ex vivo material in accordance with an embodiment of the present invention can be a film-like or membrane-like cell culture substrate or a cubic cell culture substrate containing the highly dense collagen solid described above. The ex vivo material in accordance with an embodiment of the present invention makes it possible to effectively culture cells on the ex vivo material.

To the biomaterial and the ex vivo material in accordance with an embodiment of the present invention, components other than the collagen solid can be added. These components are not particularly limited. Examples of these components include elements (e.g., calcium, magnesium, potassium, sodium, chloride, zinc, iron, and copper, or ions thereof), inorganic acids (phosphoric acid, acetic acid, and carbonic acid, or ions thereof), organic acids (pyruvic acid, acetyl-CoA, citric acid, oxalacetic acid, succinic acid, and fumaric acid, or ions thereof), low molecular weight compounds (e.g., CaCO₃), nucleic acids (DNA, RNA, plasmids), nucleosides, nucleotides, ATP, GTP, NADH, FADH₂, siRNA, miRNA, lipids, amino acids, proteins, cytokines, growth factors (e.g., FGF, bFGF, VEGF, BMP, TGF-β, PDGF, HGF, and IGF), monosaccharides (glucose, fucose, glucosamine), polysaccharides (hyaluronic acid, trehalose, amylose, pectin, cellulose, glycogen, starch, and chitin), chemically synthesized drugs, natural drugs, enzymes, hormones (testosterone, dihydrotestosterone, estrone, estradiol, progesterone, luteinizing hormone, follicle-stimulating hormone, thyroid hormone), antibiotics, anticancer drugs, proteoglycans, antibodies, exosomes, and cytoclastic components, mixtures thereof, and the like.

Each of the biomaterial and the ex vivo material in accordance with an embodiment of the present invention can contain the collagen solid in an amount of preferably 0.1% by weight to 100% by weight, more preferably 50% by weight to 100% by weight, more preferably 90% by weight to 100% by weight, more preferably 95% by weight to 100% by weight, most preferably 100% by weight. In the biomaterial in accordance with an embodiment of the present invention, components other than the collagen solid can be contained in a total amount of 0% by weight to 99.9% by weight, 0% by weight to 50% by weight, 0% by weight to 10% by weight, 0% by weight to 5% by weight, or 0% by weight.

A method of using the biomaterial obtained as described above can include, for example, (i) a cleaning step of cleaning and sterilizing a biomaterial containing a collagen solid, (ii) an implantation step of implanting the cleaned biomaterial into a biological tissue of interest, and (iii) an evaluation step of evaluating a degree of progression of bone adhesion at a site where the biomaterial has been implanted in the biological tissue.

Examples of the cleaning step include cleaning of the biomaterial with an organic solvent (70% ethanol, acetone, or the like), cleaning of the biomaterial with sterile water, and sterilization of the biomaterial by UV-irradiation. Examples of the implantation step include a process of implanting the biomaterial into a biological tissue of interest, and a process of filling a biological tissue of interest with the biomaterial. Examples of the evaluation step include evaluation with medical imaging technology, mechanical evaluation, immunohistochemical evaluation (i.e., evaluation by immunostaining) and histological evaluation.

In the evaluation with medical imaging technology, for example, images of the site at which the biomaterial has been implanted are obtained by radiography or computed tomography, the images are classified in accordance with predetermined evaluation criteria, and a degree of repair is evaluated based on the classification.

In the mechanical evaluation, for example, mechanical strength of the site at which the biomaterial has been implanted is obtained by a three-point bending extrusion tester, the images are classified in accordance with predetermined evaluation criteria, and a degree of repair is evaluated based on the classification.

In the immunohistochemical evaluation (i.e., evaluation by immunostaining), a target antigen is detected with use of a specific antibody in order to visualize the presence and localization of a component of interest on the tissue with a microscope. For example, an amount of presence at the site at which the biomaterial has been implanted is obtained with an anti-osteocalcin antibody, the images are classified in accordance with predetermined evaluation criteria, and a degree of repair is evaluated based on the classification. In regard to the antibody, a plurality of antibodies can be used together or individually in accordance with a degree of maturity of a bone regeneration tissue. Note, however, that the present invention is not limited to this.

In the histological evaluation, images of the site at which the biomaterial has been implanted are obtained after HE stain or SO stain, the images are classified in accordance with predetermined evaluation criteria, and a degree of repair is evaluated based on the classification.

A method of using the ex vivo material obtained as described above can include, for example,
(i) a cleaning step of cleaning and sterilizing an ex vivo material containing the collagen solid:
(ii) a shaping step of forming the cleaned ex vivo material into a shape for intended cell culture;
(iii) a culture step of seeding and culturing cells on the shaped ex vivo material; and (iv) an evaluation step of evaluating morphology and function of cells.

Examples of the cleaning step include cleaning of the ex vivo material with an organic solvent (70% ethanol, acetone, and the like), cleaning of the ex vivo material with sterile water, and sterilization of the ex vivo material by UV-irradiation. Examples of the shaping step include a process of injecting the ex vivo material into an appropriate template to obtain an intended shaped product, a process of filling the template with the ex vivo material, and a process of freeze-drying the ex vivo material. Examples of the evaluation step include image evaluation of cell morphology, moving speed evaluation, immunohistochemical evaluation (i.e., evaluation by immunostaining), evaluation of protein expression level, and evaluation of gene expression level. In the culture step, a culture medium, a culture temperature, and the like can be set in accordance with cells to be cultured, and the culture step can be carried out according to a known method.

### Examples

### [Example 1. Preparation of the columnar collagen solid]

Using actinidain, which is a cysteine protease, a degradation process was carried out on type I collagen derived from a pig at 20°C for 7 days. Thus, a collagen-cysteine protease degradation product "LASCol" (low adhesive scaffold collagen (type I)) in accordance with an aspect of the present invention having a triple-stranded structure was obtained. The LASCol was dialyzed with respect to 10 million-fold ultrapure water to remove impurities and the like, and then the solution after dialysis was placed in an appropriate container and frozen in an ultracold freezer at -80°C. After that, the LASCol was freeze-dried in a freeze dryer (FDU-2200 available from Tokyo Rikakikai Co, Ltd.)

Ultrapure water was then added to 100 mg of the freeze-dried LASCol such that the LASCol is contained at predetermined concentrations (i.e., 10 mg/mL, 30 mg/mL, 50 mg/mL, 100 mg/mL, 150 mg/mL, 180 mg/mL), and solutions thus obtained were left to stand for 3 days to 10 days in a refrigerator at 0°C to 10°C. During the above periods, the solutions were gently mixed without being bubbled, and the freeze-dried LASCol was thus completely dissolved.

Each of the solutions in which the LASCol was completely dissolved was put into a columnar template (diameter: 2.5 mm to 3.0 mm, length: 4 mm to 7 mm) and frozen at -80°C. The frozen product was placed in a chamber of the freeze dryer (FDU-2200 available from Tokyo Rikakikai Co, Ltd.) to completely remove water from the completely frozen solution by sublimation under conditions of approximately -85°C and 2.0 Pa to 2.5 Pa. Then, a LASCol solid obtained after freeze drying was taken out from the columnar template to obtain a columnar LASCol solid.

As a comparative example, commercially available pepsin-treated type I collagen (Cellmatrix Type I-C, available from Nitta Gelatin Inc.) (in other words, "atelocollagen" in which collagen was degraded with pepsin) was used, and a columnar atelocollagen solid was prepared in a manner similar to that described above. Note that ultrapure water was added to atelocollagen so as to obtain a solution having a high atelocollagen concentration. However, only a solution having an atelocollagen concentration of 18 mg/mL or 20 mg/mL was obtained. In other words, it was not physically possible to obtain a solution having an atelocollagen concentration of more than 20 mg/mL in a state in which atelocollagen was completely dissolved.

### [Example 2. Scanning electron microscopy (SEM) observation]

A razor was used to cut the columnar LASCol solid or the columnar atelocollagen solid in half in a longitudinal direction. Then, a piece of the columnar LASCol solid or the columnar atelocollagen solid was stuck on a sample table for SEM with a carbon double-faced tape for SEM so that a cut surface faced upward. Next, platinum-palladium was vapor-deposited to have a thickness of 5 nm on the cut surface with a vapor deposition device (Ion Sputter E-1030, available from Hitachi High-Technologies Corporation) to obtain an observation sample.

The observation sample was imaged using a scanning electron microscope (SU3500, Hitachi High-Technologies Corporation) (acceleration voltage: 5 kV, spot intensity: 30). The results are shown in Figs. 1 through 3.
Figs. 1 and 2 show cross-sectional images of LASCol, where collagen densities are (a) 10 mg/cm³, (b) 53 mg/cm³, (c) 81 mg/cm³, (d) 150 mg/cm³, (e) 209 mg/cm³, and (f) 264 mg/cm³.
Fig. 3 shows cross-sectional images of the atelocollagen solid of Comparative Example, where collagen densities are (a) 18 mg/cm³ and (b) 26 mg/cm³.

From Figs. 1 through 3, it can be seen that the LASCol solids are more densely packed with the degradation product, as compared with the atelocollagen solids. From Figs. 1 and 2, it was observed that, when the concentration of collagen dissolved in the solvent increases, the inside of the LASCol solid became denser.

### [Example 3. Preparation of bellows-shaped LASCol solid]

In a manner similar to that of Example 1 above, ultrapure water was added to freeze-dried LASCol so that a concentration of the LASCol became 100 mg/mL, and the LASCol was completely dissolved. A solution in which the LASCol was completely dissolved was put into a bellows-shaped bent tubular template 1 (root diameter (D1): 4.5 mm, outer diameter (D2): 5.1 mm, length (L1): 20.1 mm, (L2): 16.5 mm) or a bellows-shaped stretched tubular template 2 (root diameter (D1): 4.5 mm, outer diameter (D2): 5.1 mm, length (L3): 27.5 mm) and was frozen at -80°C.

With use of the freeze dryer (FDU-2200 available from Tokyo Rikakikai Co, Ltd.), water was completely removed from the completely frozen solution by sublimation under conditions of approximately -85°C and 2.0 Pa to 2.5 Pa. Then, a LASCol solid obtained after freeze drying was taken out from the tubular template 1 or the tubular template 2 to obtain a bellows-shaped LASCol solid. Fig. 4 shows images of the bellows-shaped LASCol solids.

(a) of Fig. 4 shows a bent bellows-shaped LASCol solid. (b) of Fig. 4 shows a stretched bellows-shaped LASCol solid. It can be seen that the LASCol in accordance with an embodiment of the present invention can be formed into intended shapes.

### [Example 4. Preparation of small-diameter-tubular LASCol solid]

In a manner similar to that of Example 1 above, freeze-dried LASCol was completely dissolved in ultrapure water so that a concentration of the LASCol became 100 mg/mL or 150 mg/mL. A solution in which the LASCol was completely dissolved was put into a tubular template 3 (inner diameter (D1): 4.0 mm, outer diameter (D2): 6.7 mm, length (L4): 7.5 mm), a tubular template 4 (inner diameter (D1): 2.2 mm, outer diameter (D2): 3.4 mm, length (L5): 29.1 mm), or a tubular template 5 (inner diameter (D1): 0.95 mm, outer diameter (D2): 2.50 mm, length (L6): 24.5 mm), and was frozen at -80°C.

With use of the freeze dryer (FDU-2200 available from Tokyo Rikakikai Co, Ltd.), water was completely removed from the completely frozen solution by sublimation under conditions of approximately -85°C and 2.0 Pa to 2.5 Pa. Then, a LASCol solid obtained after freeze drying was taken out from the tubular template 3, the tubular template 4, or the tubular template 5 to obtain a small-diameter-tubular LASCol solid. Fig. 5 shows images of the small-diameter-tubular LASCol solids.

(a) of Fig. 5 shows a small-diameter-tubular LASCol solid obtained by filling the tubular template 3 with a solution containing LASCol at a collagen concentration of 100 mg/mL. (b) of Fig. 5 shows a small-diameter-tubular LASCol solid obtained by filling the tubular template 4 with a solution containing LASCol at a collagen concentration of 150 mg/mL. (c) of Fig. 5 shows a small-diameter-tubular LASCol solid obtained by filling the tubular template 5 with a solution containing LASCol at a collagen concentration of 150 mg/mL.

From (a) through (c) of Fig. 5, it can be seen that, at any collagen concentration in LASCol, small-diameter-tubular LASCol solids formed into a very small hollow tubular shape are obtained.

### [Example 5. Strength test of columnar LASCol solid]

In a manner similar to that of Example 1, a columnar LASCol solid (diameter: 2.5 mm) was obtained by freeze-drying a solution containing LASCol having a predetermined collagen concentration (30 mg/mL, 50 mg/mL, 100 mg/mL, 150 mg/mL, 180 mg/mL). Similarly, a columnar atelocollagen solid (diameter: 2.5 mm) was obtained by freeze-drying a solution containing atelocollagen at a predetermined collagen concentration (20 mg/mL). The columnar LASCol solids and the columnar atelocollagen solid were cut by a razor to obtain columnar pieces having a diameter of 5 mm and a length of 5 mm.

Strength tests of the columnar LASCol solids and the columnar atelocollagen solid were carried out using a small tabletop tester EZ TEST device (Force Transducer SM-100N-168, available from Shimadzu Corporation). A longitudinal axis of each of the columnar LASCol solid pieces and the columnar atelocollagen solid piece was aligned vertically. Each of the pieces was set so that the longitudinal axis was perpendicular to a pressurizing unit of the small tabletop tester EZ TEST device. Then, a stress-strain curve of each of the pieces was measured by a conventional method. Then, from inclinations of the curves, tangent moduli of the columnar LASCol solids and the columnar atelocollagen solid were calculated.

Densities of the columnar LASCol solids and the columnar atelocollagen solid were measured according to the following method. That is, with use of a standard digital caliper (Digimatic caliper CD-10AX (product number) available from Mitutoyo Corporation), a diameter (mm) and a length (mm) of the columnar shaped product were measured, and a volume (cm³) of the cylinder was calculated from a radius, the length, and the circular constant. In addition, a weight (mg) of the columnar solid was measured using a semi-microelectronic analytical balance (LIBROR AEL-40SM (product number) available from Shimadzu Corporation). The weight (mg) was divided by the volume (cm³) to calculate the density (mg/cm³).

Table 1 indicates collagen concentrations of solutions used in preparing the columnar LASCol solids and the columnar atelocollagen solid and densities and tangent moduli of the LASCol solids and the atelocollagen solid after freeze drying.

From Table 1, it can be seen that the use of LASCol makes it possible to obtain the LASCol solid having a large density and a large tangent modulus. In contrast, it can be seen that, when atelocollagen is used, only an atelocollagen solid having a small density and a small tangent modulus can be obtained.

**[Table 1]**

| | Before freeze drying (Collagen solution) | After freeze drying (LASCol solid/ atelocollagen solid) | |
|---|---|---|---|
| | Concentration (mg/mL) | Density (mg/cm³) | Tangent modulus (kPa) |
| Collagen-cysteine protease degradation product "LASCol" | 180 | 264 | 30,277 |
| | 150 | 209 | 9,465 |
| | 100 | 150 | 304 |
| | 50 | 81 | 175 |
| | 30 | 53 | 93 |
| Collagen-pepsin degradation product "Atelocollagen" | 20 | 26 | 29 |

### [Example 6. SEM-EDX analysis of Ca²⁺, Na⁺, Cl⁻-impregnated columnar LASCol solid]

In a manner similar to that of Example 1 above, LASCol was freeze-dried. The freeze-dried LASCol was then added to ultrapure water containing 5 mM of Ca²⁺, 4 mM of Na⁺, and 15 mM of Cl⁻ so that a final collagen concentration became 50 mg/mL. Solutions thus obtained were then left to stand in a refrigerator at 0°C to 10°C for 3 days to 10 days. During the above periods, the solutions were gently mixed without being bubbled, and the freeze-dried LASCol was thus completely dissolved.

The solution in which the LASCol was completely dissolved was put into a columnar template (diameter: 3.5 mm, length: 2 mm to 5 mm) and was freeze-dried in a manner similar to that of Example 1. A LASCol solid was then taken out from the columnar template to obtain a Ca²⁺, Na⁺, Cl⁻-impregnated columnar LASCol solid.

As a comparative example, a Ca²⁺, Na⁺, Cl⁻-non-impregnated columnar LASCol solid was prepared in a similar manner, except that ultrapure water which did not contain Ca²⁺, Na⁺, and Cl⁻ was used instead of the ultrapure water containing 5 mM of Ca²⁺, 4 mM of Na⁺, and 15 mM of Cl⁻.

Then, cut surfaces of the Ca²⁺, Na⁺, Cl⁻-impregnated columnar LASCol solid and cut surfaces of the Ca²⁺, Na⁺, Cl⁻-non-impregnated columnar LASCol solid were imaged by a scanning electron microscope (SU3500 available from Hitachi High-Technologies Corporation) (acceleration voltage: 15 kV, spot intensity: 60) in a manner similar to that of Example 2. In addition, a scanning electron microscope/energy dispersive x-ray spectroscope (SEM-EDX, OCTANE PRIME (model number) available from EDAX Japan) was used to detect element ions contained in the Ca²⁺, Na⁺, Cl⁻-impregnated columnar LASCol solid and the Ca²⁺, Na⁺, Cl⁻-non-impregnated columnar LASCol solid. The results are shown in (a) through (d) of Fig. 6 and Table 2.

(a) of Fig. 6 shows a cross-sectional image along a longitudinal axis of the Ca²⁺, Na⁺, Cl⁻-non-impregnated columnar LASCol solid. (b) of Fig. 6 shows a result of SEM-EDX analysis of the cross section along the longitudinal axis of the Ca²⁺, Na⁺, Cl⁻-non-impregnated columnar LASCol solid. (c) of Fig. 6 shows a cross-sectional image along a lateral axis of the Ca²⁺, Na⁺, Cl⁻-impregnated columnar LASCol solid. (d) of Fig. 6 shows a result of SEM-EDX analysis of the cross section (in particular, an area surrounded by the square in (c) of Fig. 6) along the lateral axis of the Ca²⁺, Na⁺, Cl⁻-impregnated columnar LASCol solid. Table 2 shows data obtained by quantifying amounts of element ions detected in (d) of Fig. 6. The LASCol solid was proved to contain all the elements of Na, CI, and Ca.

**[Table 2]**

| Signal intensity | NaK | PtM | ClK | CaK |
|---|---|---|---|---|
| Value | 2.52 | 20.48 | 9.21 | 3.23 |

(a) through (d) of Fig. 6 and Table 2 reveal that a plurality of various substance components, notably Ca²⁺, Na⁺, Cl⁻, can be contained in the LASCol solid in accordance with an embodiment of the present invention at any collagen concentration. In other words, by mixing ultrapure water with optional substance components with which the solid is to be impregnated prior to freeze drying, a substance-impregnated collagen solid can be easily prepared.

### [Example 7. SEM-EDX analysis-2 of Ca²⁺-impregnated columnar LASCol solid]

A LASCol solid was prepared in a manner similar to that of Example 1, except that ultrapure water was added so that a concentration of LASCol became 50 mg/mL instead of 100 mg. After freeze drying, the LASCol solid was taken out from a columnar template (diameter: 3.5 mm, length: 10 mm) to obtain a columnar LASCol solid. The LASCol solid was immersed in ultrapure water which contained 10 mM of Ca²⁺ and was kept at 37°C for 15 minutes while keeping warm, and then the LASCol solid was freeze-dried again to prepare a Ca²⁺-impregnated columnar LASCol solid.

The Ca²⁺-impregnated columnar LASCol solid thus obtained was observed by scanning electron microscopy and analyzed by SEM-EDX in a manner similar to that of Example 6. The results are shown in (e) and (f) of Fig. 6 and Table 3. (e) of Fig. 6 shows an image of an outer surface of the Ca²⁺-impregnated columnar LASCol solid. (f) of Fig. 6 shows a result of SEM-EDX analysis of the outer surface of the Ca²⁺-impregnated columnar LASCol solid. Table 3 shows data obtained by quantifying amounts of element ions detected in (f) of Fig. 6.

**[Table 3]**

| Signal intensity | CK | NK | OK | CaK |
|---|---|---|---|---|
| Value | 132.44 | 4.69 | 33.73 | 8.01 |

(e) and (f) of Fig. 6 and Table 3 reveal that any of various substances, notably Ca²⁺, can be contained in the LASCol solid in accordance with an embodiment of the present invention. That is, it was possible to prepare the LASCol solid containing an intended substance component by taking out a LASCol solid having an arbitrary shape and an arbitrary density after freeze drying, and then immersing the LASCol solid in an appropriate solution in which the intended substance component with which the sold was to be impregnated was dissolved at an arbitrary concentration. The compound-impregnated LASCol solid in accordance with an embodiment of the present invention does not change the structure and properties of LASCol contained in the solid, and therefore the compound-impregnated LASCol solid can be used even in vivo. Moreover, in Example 7, the LASCol solid is not dissolved in the solution containing the optional substance component with which the solid is to be impregnated. Therefore, change in solubility of the substance component with respect to the solution due to dissolution of the LASCol solid in the solution can be ignored. Furthermore, a LASCol solid containing a plurality of intended substance components can be prepared as follows: one of the plurality of substance components is dissolved simultaneously with LASCol in the same solution, and a mixture thus obtained is freeze-dried; and then a LASCol solid containing the substance component is immersed in an appropriate solution in which the other substance component has been dissolved. In this Example, element ions contained in the LASCol solid in accordance with the present invention were quantified without destroying the LASCol solid. Note that, in the present invention, the substance with which the solid is to be impregnated is not particularly limited and can be any substance.

### [Example 8. Columnar LASCol solid having small holes]

A columnar LASCol solid was prepared in a manner similar to that of Example 1, except that 100 mg of the freeze-dried LASCol described above was added to ultrapure water so that a collagen concentration became 100 mg/mL, and the above columnar template (diameter: 2.5 mm to 3.0 mm, length: 4 mm to 7 mm) was changed to another columnar template (diameter: 3.5 mm, length: 2 mm to 5 mm). Three through holes were formed in the obtained columnar LASCol solid using a needle bar (outer diameter: 200 µm) to obtain an observation sample. The observation sample was imaged with a stereoscopic microscope (SZ61, available from Olympus Corporation). The results are shown in Fig. 7.

(a) of Fig. 7 is an image of the columnar LASCol solid taken with top illumination. (b) of Fig. 7 is an image of the columnar LASCol solid taken with bottom illumination. The three through holes were maintained with little deformation. In contrast, the columnar atelocollagen solid (diameter: 3.5 mm, length: 2 mm to 5 mm) which was prepared in a manner similar to that of Example 1 and had a collagen concentration of 20 mg/mL could not have through holes (not shown). This is because the columnar atelocollagen solid has low mechanical strength and many voids and therefore, even though holes are formed, shapes of the holes cannot be kept unchanged or the holes are closed. The collagen solid in accordance with an embodiment of the present invention can have small holes in any size and in any number.

### [Example 9. SEM-EDX analysis-3 of Ca²⁺-impregnated columnar LASCol solid]

Ca²⁺-impregnated columnar LASCol solids having different molarities of Ca²⁺ were obtained by taking out freeze-dried LASCol solids from columnar templates (diameter: 3.5 mm, length: 5 mm to 10 mm) in a manner similar to that of Example 6, except that 100 mg of LASCol was added to ultrapure water containing 10 mM of Ca²⁺ or 20 mM of Ca²⁺ so that a final collagen concentration became 50 mg/mL, and 100 mg of LASCol was added to ultrapure water containing 5 mM of Ca²⁺ or 10 mM of Ca²⁺ so that a final collagen concentration became 100 mg/mL.

Then, insides of the Ca²⁺-impregnated columnar LASCol solids having different molarities of Ca²⁺ were imaged by a scanning electron microscope (SU3500 available from Hitachi High-Technologies Corporation) (acceleration voltage: 15 kV, spot intensity: 60) in a manner similar to that of Example 2. In addition, a scanning electron microscope/energy dispersive x-ray spectroscope (SEM-EDX, OCTANE PRIME (model number) available from EDAX Japan) was used to detect element ions contained in the Ca²⁺-impregnated columnar LASCol solids having different molarities of Ca²⁺. The results are shown in (a) through (h) of Fig. 8 and Table 4.

(a) of Fig. 8 shows an image of the 10 mM Ca²⁺-impregnated columnar LASCol solid (final collagen concentration: 50 mg/mL). (b) of Fig. 8 shows the result of SEM-EDX analysis in a whole screen area of (a) of Fig. 8. (c) of Fig. 8 shows an image of the 20 mM Ca²⁺-impregnated columnar LASCol solid (final collagen concentration: 50 mg/mL). (d) of Fig. 8 shows the result of SEM-EDX analysis in a whole screen area of (c) of Fig. 8. (e) of Fig. 8 shows an image of the 5 mM Ca²⁺-impregnated columnar LASCol solid (final collagen concentration: 100 mg/mL). (f) of Fig. 8 shows the result of SEM-EDX analysis in a whole screen area of (e) of Fig. 8. (g) of Fig. 8 shows an image of the 10 mM Ca²⁺-impregnated columnar LASCol solid (final collagen concentration: 100 mg/mL). (h) of Fig. 8 shows the result of SEM-EDX analysis in a whole screen area of (g) of Fig. 8.

Table 4 shows data obtained by quantifying intensity of element ions detected in Fig. 8. In the LASCol solid prepared using the degradation product having a collagen concentration of 50 mg/mL, CaK/NK is a numerical value calculated by setting the intensity of N (NK) as a denominator and the intensity of Ca (CaK) as a numerator. Assuming that a value of CaK/NK was 1 when 10 mM of Ca²⁺ was used, a value of CaK/NK was 2.0 when 20 mM of Ca²⁺ was used. In addition, in the LASCol solid prepared using the degradation product having a collagen concentration of 100 mg/mL, CaK/NK was similarly calculated. Assuming that a value of CaK/NK was 1 when 5 mM of Ca²⁺ was used, a value of CaK/NK was 1.8 when 10 mM of Ca²⁺ was used. That is, when the molarity of Ca²⁺ with which the solid was impregnated in the solvent was doubled, the value of CaK/NK of the Ca²⁺-impregnated columnar LASCol solid was almost doubled. In other words, it has been found that the intensity (amount) of Ca detected in the LASCol solid was increased in proportion to the molar ratio of an optional substance (e.g., Ca²⁺) in ultrapure water containing the optional substance (e.g., Ca²⁺) used in preparing the Ca²⁺-impregnated columnar LASCol solid. Therefore, it was proved that the Ca²⁺-impregnated columnar LASCol solid contained Ca in an amount depending on the molarity of Ca²⁺ contained in the ultrapure water when the LASCol solid was prepared.

**[Table 4]**

| Signal intensity | 50 mg/mL LASCol | | 100 mg/mL LASCol | |
|---|---|---|---|---|
| | 10 mM Ca²⁺ | 20 mM Ca²⁺ | 5 mM Ca²⁺ | 10 mM Ca²⁺ |
| NK | 10.33 | 11.29 | 14.51 | 12.73 |
| CaK | 4.12 | 9.21 | 1.83 | 2.81 |
| CaK/NK | 0.399 | 0.816 | 0.126 | 0.221 |
| CaK/NK ratio | 1 | 2 | - | - |
| | - | - | 1 | 1.8 |

From (a) through (h) of Fig. 8 and Table 4, it has been found that the LASCol solid in accordance with an embodiment of the present invention can contain Ca²⁺ while increasing or decreasing an intended amount of Ca²⁺. In other words, by mixing, at any concentration, ultrapure water with an optional substance component with which the solid is to be impregnated prior to freeze drying, a collagen solid can be easily prepared which is impregnated with the intended substance component in a necessary amount.

### [Example 10. Evaluation of bone regenerative ability in animal experiment]

### [10-1. Preparation of columnar LASCol solid for implantation]

A solution containing LASCol was sterilized by filtration and freeze drying before being formed into a columnar shape. Then, columnar LASCol solids were prepared by freeze-drying solutions containing the LASCol after sterilization at predetermined collagen concentrations (50 mg/mL, 100 mg/mL, and 150 mg/mL) in a manner similar to that of Example 5 above. A shape of each of the columnar LASCol solids was the same as a shape of a femur having a 1 mm of defect, which will be described later. Then, the columnar LASCol solids were used in Examples described later.

### [10-2. Animal]

In this Example, rats were used. All rats used were kept in a 12-hour light-dark cycles at 25°C and in a pathogen-free state and were allowed free access to feed and water. All animal experiments were conducted according to the experimental guidelines of Kobe University School of Medicine.

### [10-3. Preparation of femur defect rat model]

After general anesthesia of the rat and exposure of a femur, two threaded K-wires were inserted into a proximal site and a distal site of the femur, respectively, and the K-wires were connected to each other by an external fixator. Subsequently, a bone was resected by a width of 1 mm using a small bone saw in the middle of the shaft of femur to prepare a femur 1 mm defect rat model. Columnar LASCol solids obtained by freeze-drying solutions containing the predetermined concentrations (50 mg/mL, 100 mg/mL, and 150 mg/mL) of LASCol were then implanted into the femur 1 mm defect rat models, respectively, followed by suturing of the surgical sites. After the rats were kept for a predetermined period of time, the bone regeneration status was evaluated.

Note that 11 cases (n=11) of populations were prepared in each of which the columnar LASCol solids were not implanted (herein referred to as "1 mm femur defect population", i.e., control). 21 cases (n=21) of populations were prepared in each of which columnar LASCol solids obtained by freeze-drying solutions containing LASCol at a collagen concentration of 50 mg/mL were implanted (herein referred to as "50 mg/mL LASCol solid implanted population"). 4 cases (n=4) of populations were prepared in each of which columnar LASCol solids obtained by freeze-drying solutions containing LASCol at a collagen concentration of 100 mg/mL were implanted (herein referred to as "100 mg/mL LASCol solid implanted population"). 7 cases (n=7) of populations were prepared in each of which columnar LASCol solids obtained by freeze-drying solutions containing LASCol at a collagen concentration of 150 mg/mL were implanted (herein referred to as "150 mg/mL LASCol solid implanted population").

(a) of Fig. 9 shows an image of preparing a 4 mm femur defect rat model by inserting threaded K-wires. (b) of Fig. 9 shows an image of implanting the columnar LASCol solid into the 4 mm femur defect rat model.

### [Example 11. Evaluation with medical imaging technology]

### [11-1. Evaluation criteria based on modified RUST score]

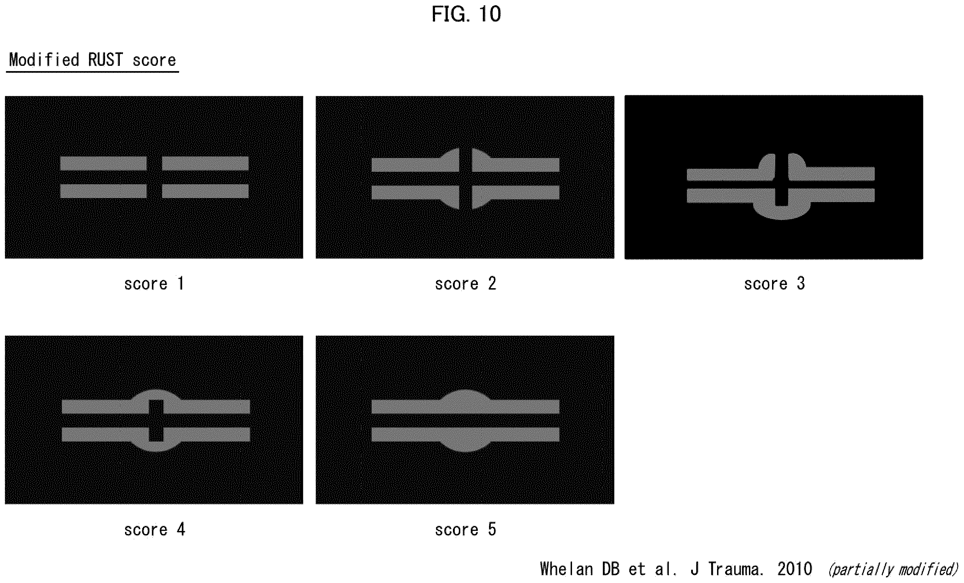

Evaluation of a degree of bone adhesion based on radiographic images (which will be described later) was conducted in accordance with modified RUST scores. Evaluation criteria in which the evaluation criteria of radiographic union scale in tibial (RUST) fracture score described in [Whelan D. B. et al. The Journal of Trauma, 2010] were partially modified were used in this test (see Fig. 10). In this test, five-level evaluation criteria, i.e., score 1 through score 5 were used. In those scores, the higher number indicates that better bone adhesion is in progress. In this Example, it was determined that "bone adhesion" occurred when the modified RUST score based on the radiographic image was evaluated to be the score 4 or 5.

### [11-2. Radiographic image evaluation]

Immediately after, 14 days after, and 28 days after implantation of the columnar LASCol solids, the populations were imaged using a radiography device (Qpix VPX-30E available from TOSHIBA).

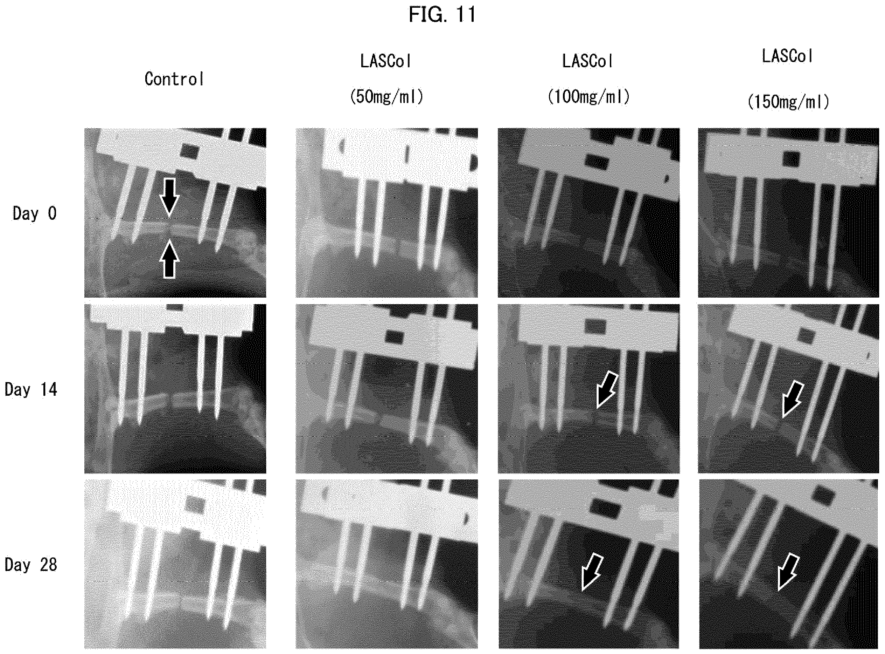

Fig. 11 shows radiographic images of the 1 mm femur defect population and the LASCol solid implanted populations taken immediately after implantation, 14 days after implantation, and 28 days after implantation.

Table 5 shows the number of individuals in which bone adhesion occurred and a calculation result of a ratio of individuals in which bone adhesion occurred in each of the populations on the 28th day after implantation.

**[Table 5]**

| | | | | |
|---|---|---|---|---|
| | 1 mm femur defect population (Control) | Population in which columnar LASCol solids were implanted into 1 mm femur defect rat models | | |
| | | 50 mg/mL LASCol solid | 100 mg/mL | 150 mg/mL |

| | | implanted population | LASCol solid implanted population | LASCol solid implanted population |
|---|---|---|---|---|
| Number of bone adhesion | 2 | 11 | 4 | 7 |
| individuals (n) | (11) | (21) | (4) | (7) |
| Bone adhesion ratio | 18% | 52% | 100% | 100% |

As shown in Fig. 11 and Table 5, in the 1 mm femur defect population, 2 of 11 cases had bone adhesion, and the ratio of bone adhesion was 18%. In the 50 mg/mL LASCol solid implanted population, 11 of 21 cases had bone adhesion, and the ratio of bone adhesion was 52%. In the 100 mg/mL LASCol solid implanted population, 4 of 4 cases had bone adhesion, and the ratio of bone adhesion was 100%. In the 150 mg/mL LASCol solid implanted population, 7 of 7 cases had bone adhesion, and the ratio of bone adhesion was 100%. That is, the 50 mg/mL, 100 mg/mL, and 150 mg/mL LASCol solid implanted populations showed that bone adhesion was in progress on the 28th day after implantation.

### [11-3. Bone adhesion evaluation based on modified RUST score]

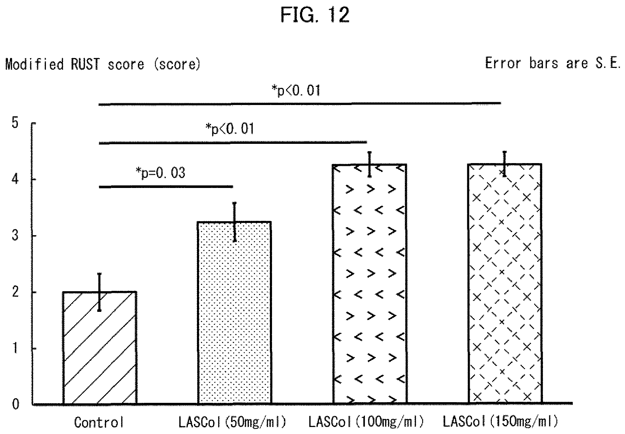

The radiographic images of the 1 mm femur defect population and the LASCol solid implanted populations taken 28 days after implantation were evaluated for bone adhesion based on the modified RUST score. Average scores of the respective populations were calculated and are shown in Fig. 12. The scores of the respective populations were also statistically evaluated (p=0.01).

As shown in Fig. 12, in the 1 mm femur defect population, the modified RUST score was approximately 2. In contrast, the 50 mg/mL, 100 mg/mL, and 150 mg/mL LASCol solid implanted populations had higher modified RUST scores than that of the 1 mm femur defect population. In addition, the modified RUST scores were higher in the LASCol solid implanted populations of 100 mg/mL and 150 mg/mL, as compared with the 50 mg/mL LASCol solid implanted population.

### [11-4. µCT image evaluation]

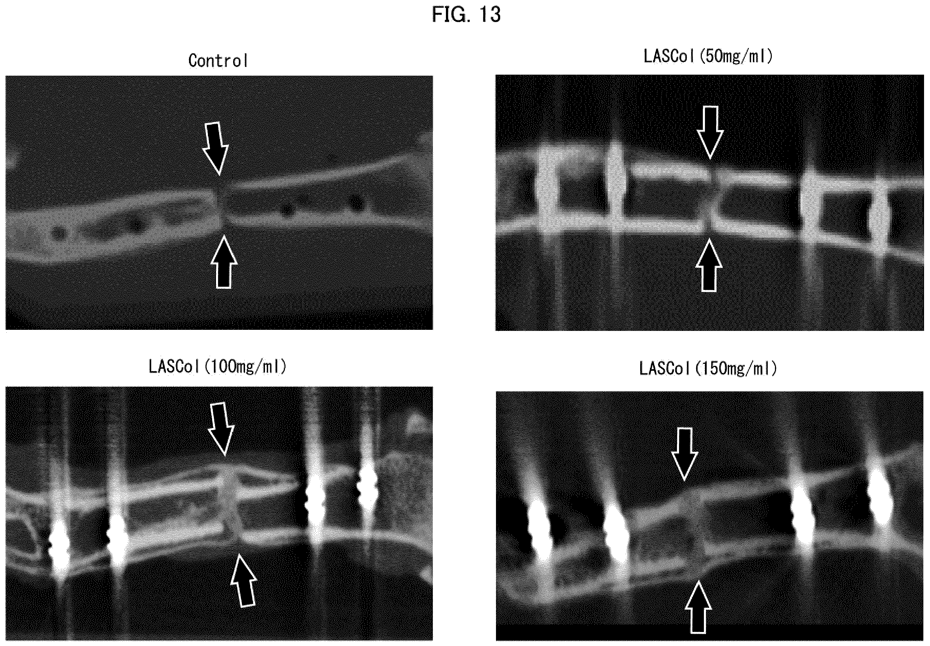

14 days and 28 days after implantation, computed tomography (CT) was carried out with a µCT device (R_mCT; Rigaku Mechatronics Co., Ltd., Tokyo, Japan) to evaluate a degree of progress of bone adhesion. Rats were euthanized by cervical dislocation and subjected to computed tomography, followed by histological evaluation as described later. The results are shown in Figs. 13 through 17.

Fig. 13 shows CT images of the 1 mm femur defect population and the LASCol solid implanted populations taken 28 days after implantation. As shown in Fig. 13, the LASCol solid implanted populations showed bone adhesion at any of the LASCol solid concentrations. In particular, complete bone adhesion was observed in the LASCol solid implanted populations of 100 mg/mL and 150 mg/mL.

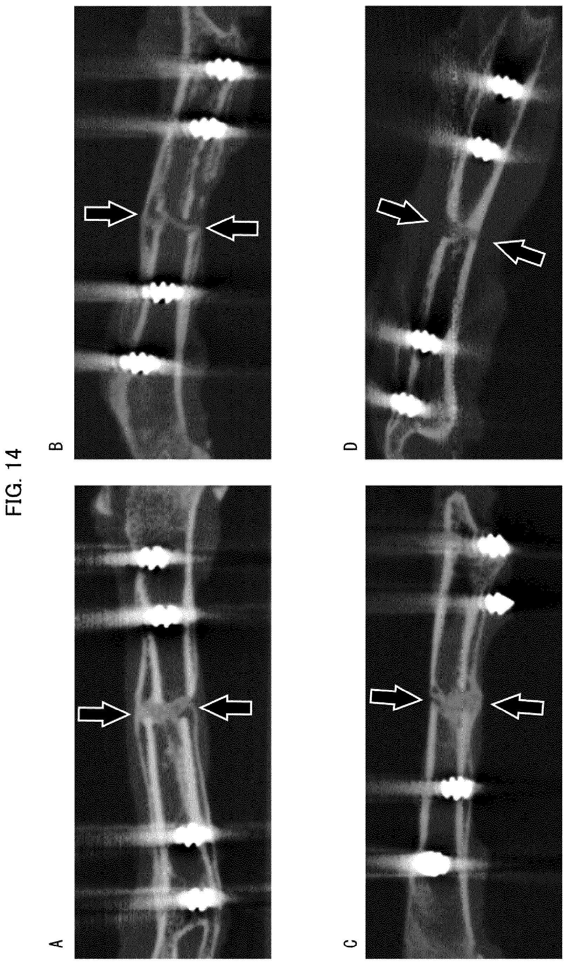

Fig. 14 shows CT images of the 100 mg/mL LASCol solid implanted population taken 28 days after implantation. A through D represent four individuals, respectively. As shown in Fig. 14, complete bone adhesion was observed in all rats.

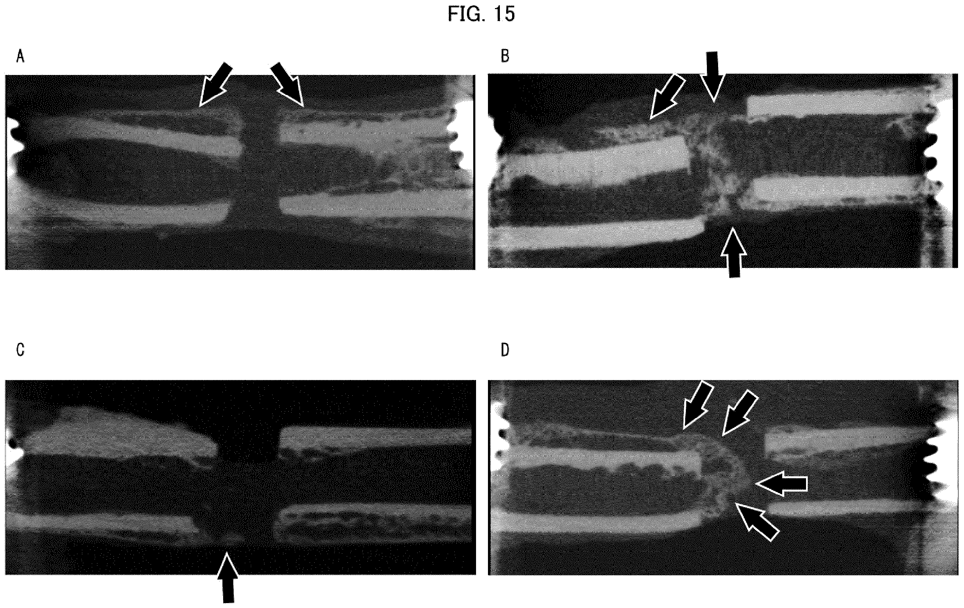

Fig. 15 shows CT images of the 150 mg/mL LASCol solid implanted population taken 14 days after implantation. A through D show images of representative four individuals of the seven individuals (n=7). It can be seen that bone tissue formation is in progress and bone regeneration is in progress particularly at the sites indicated by the arrows in the images.

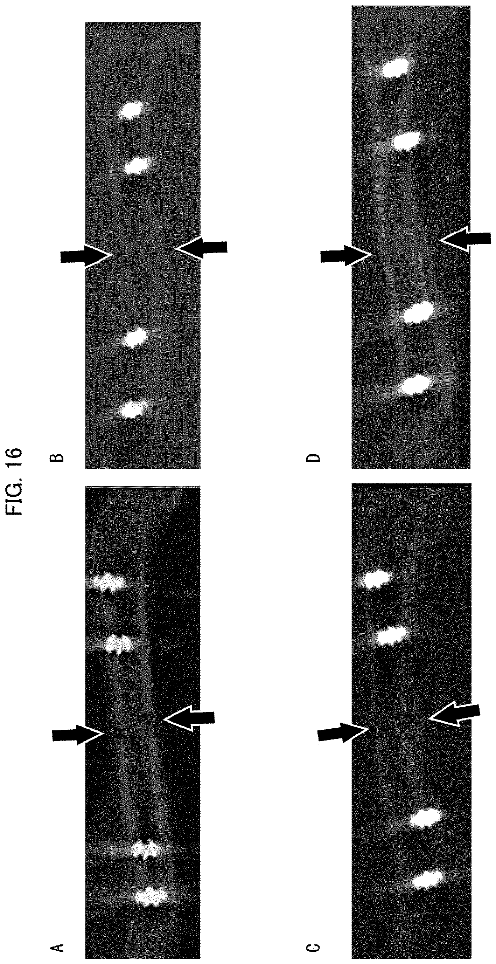

Fig. 16 shows CT images of the 150 mg/mL LASCol solid implanted population taken 28 days after implantation. A through D show images of representative four individuals of the seven individuals (n=7). It was seen that bone tissue formation progressed to achieve complete bone adhesion particularly at the sites indicated by the arrows in the images, as compared with the case on the 14th day after implantation (shown in Fig. 15).

### [Example 12. Histological Evaluation]

### [12-1. Femur extraction]

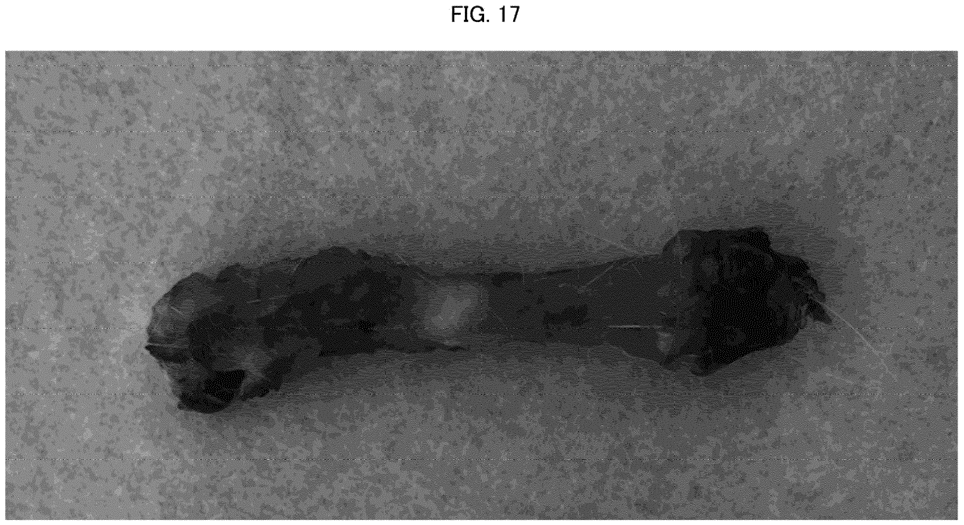

The femurs were extracted, 14 days and 28 days after implantation, from the 50 mg/mL, 100 mg/mL and the 150 mg/mL LASCol solid implanted populations which had been subjected to [11-4. µCT image evaluation] above. Fig. 17 shows an image of a femur extracted from a rat in the 150 mg/mL LASCol solid implanted population taken 28 days after implantation.

As shown in Fig. 17, bone adhesion was observed 28 days after implantation at the site where the 150 mg/mL LASCol solid was implanted in a rat having the 1 mm defect of femur.

### [12-2. Evaluation criteria based on Allen's score]

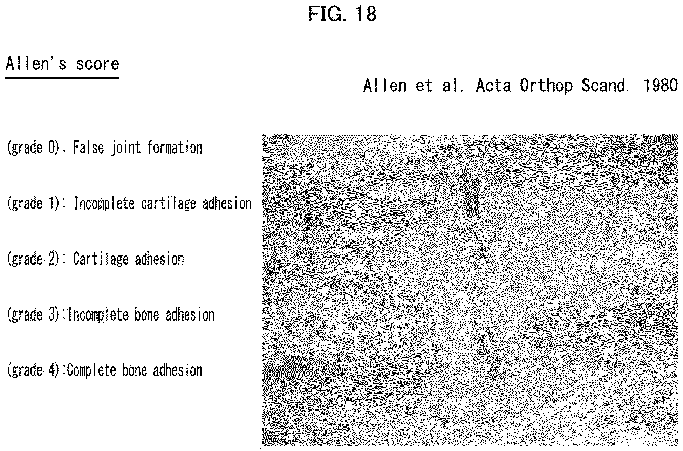

In the histological evaluation, the degree of progress of bone adhesion in stained images (described later) was evaluated in accordance with Allen's scores. The evaluation criteria were in accordance with the evaluation criteria described in [H. L. Allen et al. Acta Orthopaedica Scandinavica, 1980]. The evaluation criteria of the degree of progress of bone adhesion (herein referred to as "Allen's score") are shown in Fig. 18.

### [12-3. HE stain]

A sectioned tissue of the femur extracted in [12-1. Femur extraction] above was prepared, and hematoxylin eosin stain (HE stain) was carried out on that sectioned tissue. In the HE stain, hematoxylin used was Mayer's Hematoxylin (product number: 30002, available from MUTO PURE CHEMICALS CO., LTD.) and eosin used was Eosin Y (product number: 058-00062, available from Wako), which were used in accordance with the respective use methods to stain the sectioned tissue. The sectioned tissue after staining was observed with an optical microscope (product name: BA-X700, available from Keyence Corporation, Osaka, Japan).

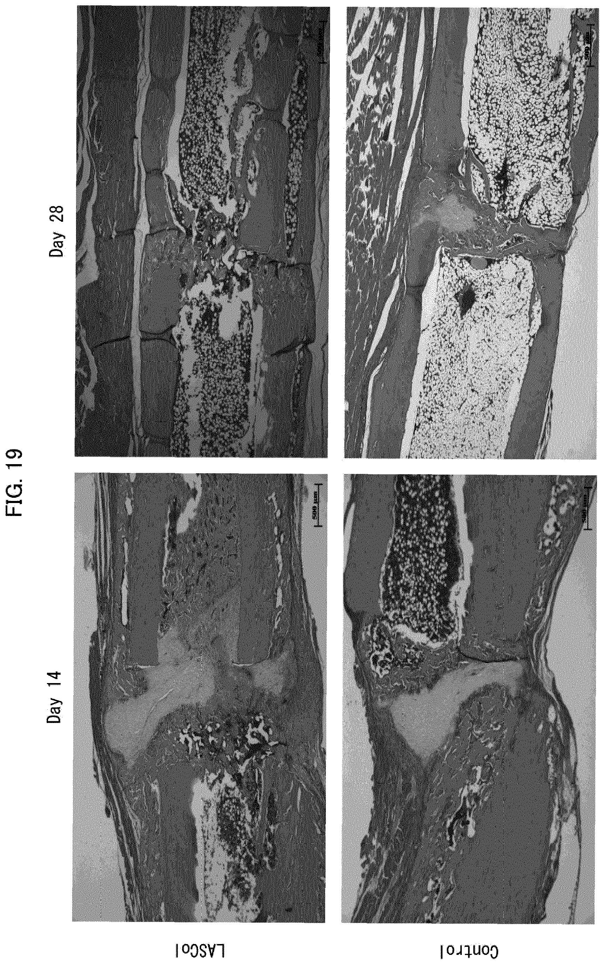

Fig. 19 shows images of the HE stained sectioned femur tissues of the 50 mg/mL LASCol solid implanted population and the HE stained sectioned femur tissues of the 1 mm femur defect population (control), which were taken 14 days and 28 days after implantation. As shown in Fig. 19, it is seen that bone adhesion was in progress on the 14th day after implantation in the femur of the 50 mg/mL LASCol solid implanted population, and bone adhesion was advancing on the 28th day after implantation from the state on the 14th day after implantation.

### [12-4. SO stain]

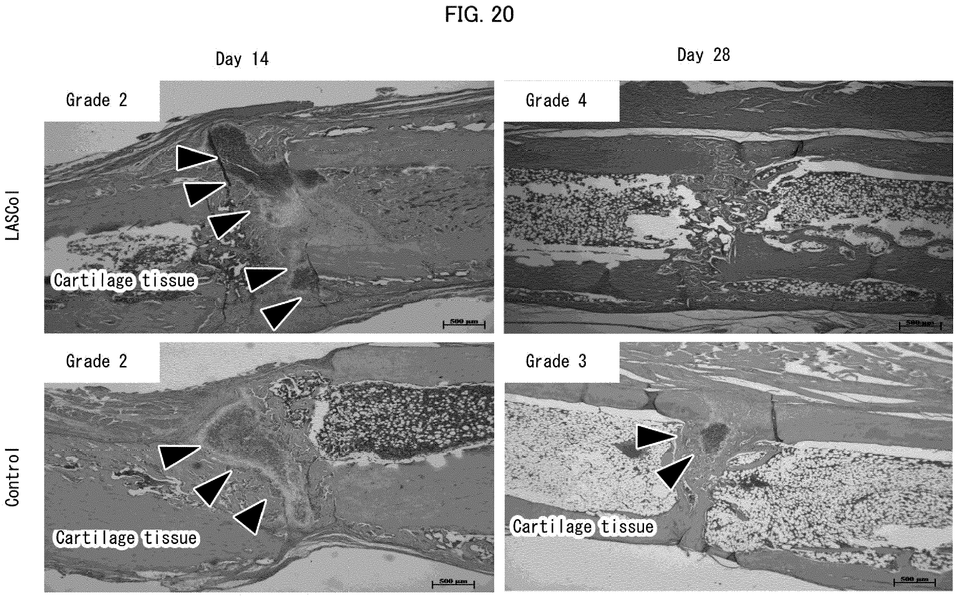

With respect to the sectioned tissue used in [12-3. HE stain] above, safranin O stain (SO stain) was carried out. In the SO stain, safranin O used was Fastgreen FCF (product number: 10720, available from CHROMA-GESELLSCHAFT) and oil red used was Basic Red2 (product number: GB01-PALO, available from Tokyo Chemical Industry Co., Ltd.), which were used in accordance with the respective use methods to stain the sectioned tissues. The sectioned tissues after staining were observed with an optical microscope (product name: BA-X700, available from Keyence Corporation, Osaka, Japan). The results are shown in Figs. 20 and 22 through 24.

Fig. 20 shows images of the SO stained sectioned femur tissues of the 50 mg/mL LASCol solid implanted individuals and the SO stained sectioned femur tissues of the 1 mm femur defect individuals (control), which were taken 14 days and 28 days after implantation. As shown in Fig. 20, in any of the sectioned femur tissues on the 14th day after implantation, formation of cartilage tissue was observed at orange-stained sites (indicated by the arrows in Fig. 20). As a result of evaluation based on the Allen's score, the evaluation scores were all Grade 2. In the 50 mg/mL LASCol solid implanted individual on the 28th day after implantation, bone adhesion progressed and the evaluation based on the Allen's score was Grade 4. In contrast, cartilage tissues (indicated by the arrows in Fig. 20) were also observed in the control 28 days after implantation but the evaluation result based on the Allen's score was Grade 3. That is, bone adhesion was incomplete.

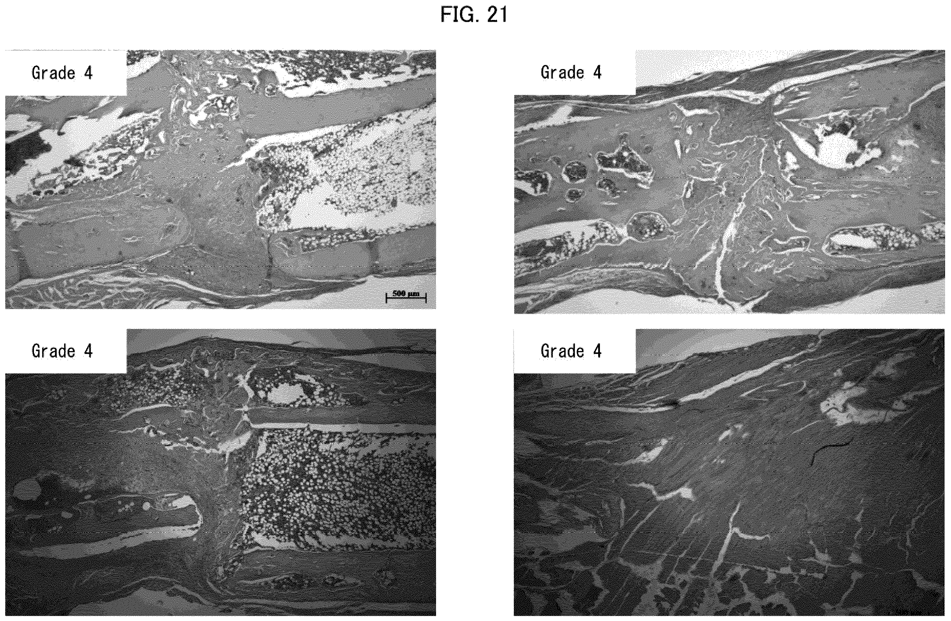

Fig. 21 shows images of sectioned femur tissues of four individuals out of the 100 mg/mL LASCol solid implanted population on the 28th day after implantation. As shown in Fig. 21, in the 100 mg/mL LASCol solid implanted population, no cartilage tissue was observed in all the individuals, and the evaluation results based on the Allen's score were all Grade 4. That is, it was found that implanting the 100 mg/mL LASCol solid into the rat resulted in complete bone adhesion on the 28th day after implantation.

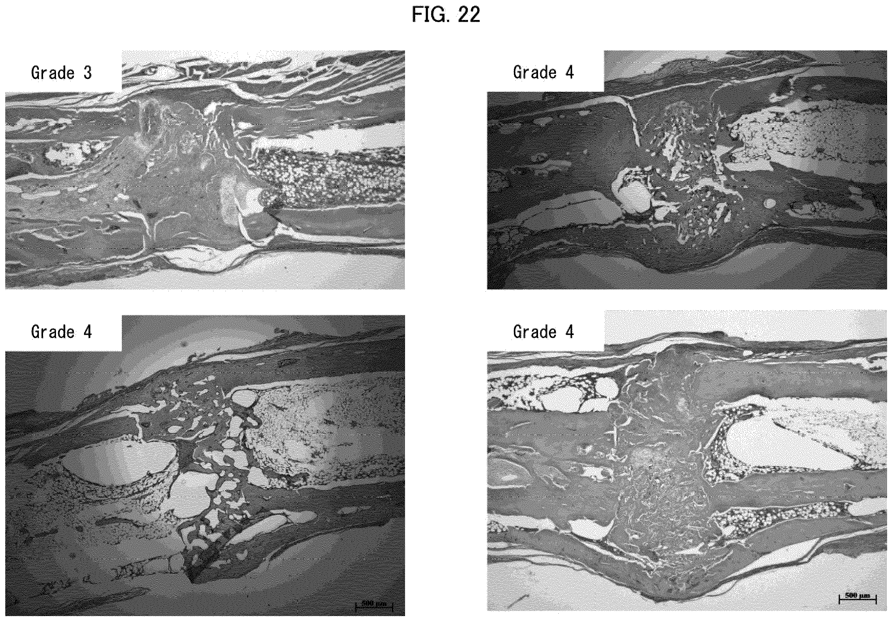

Fig. 22 shows images of sectioned femurs of four individuals out of the 150 mg/mL LASCol solid implanted population on the 28th day after implantation. As shown in Fig. 22, in the 150 mg/mL LASCol solid implanted population, the evaluation results based on the Allen's score were Grade 4 in three individuals out of four individuals (i.e., cartilage tissue was observed in one individual). That is, it was found that implanting the 150 mg/mL LASCol solid into the rat significantly promoted progress of bone adhesion on the 28th day after implantation.

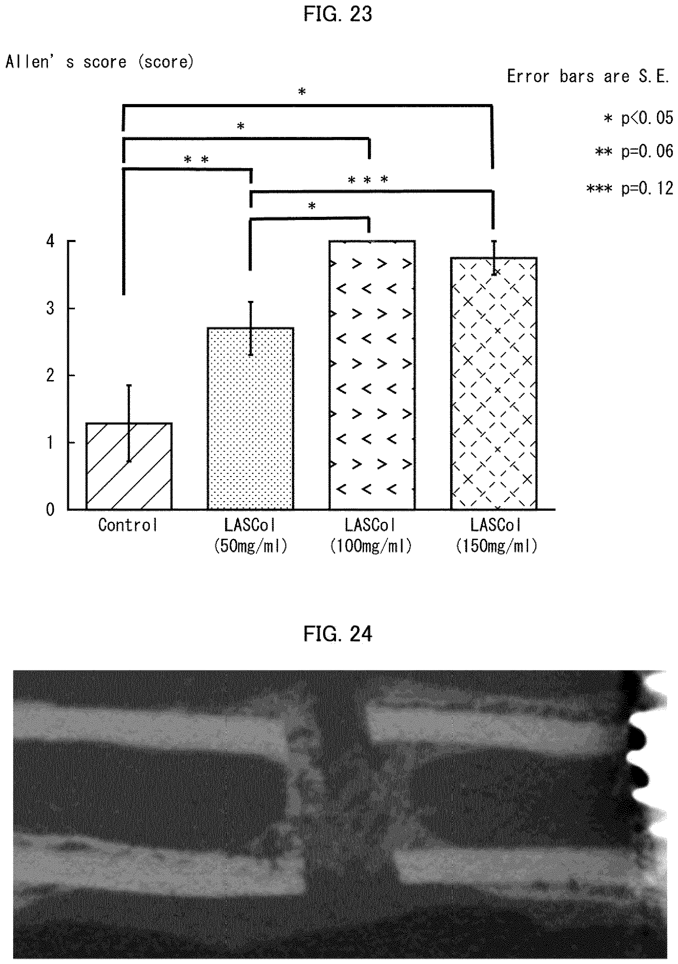

Further, Fig. 23 shows results of carrying out the above SO stain and evaluation based on the Allen's score 28 days after implantation with respect to: 10 individuals (n=10) of the 50 mg/mL LASCol solid implanted population; four individuals (n=4) of the 100 mg/mL LASCol solid implanted population; four individuals (n=4) of the 150 mg/mL LASCol solid implanted population, and seven individuals (control, n=7) of the 1 mm femur defect population. The scores for the LASCol solid implanted populations of respective concentrations and the 1 mm femur defect population were then statistically evaluated. As shown in Fig. 23, on the 28th day after implantation, the 50 mg/mL LASCol solid implanted population was found to have the higher Allen's score than the control population, and have the tendency of progressed bone adhesion (p=0.06). Moreover, the 100 mg/mL LASCol solid implanted population and the 150 mg/mL LASCol solid implanted population had significantly higher Allen's scores and had progressed bone regeneration, as compared with the control population (p<0.05).

### [Example 13. CaCO₃-containing LASCol solid]

Elements constituting hard tissues such as calcium and phosphorus have a function of promoting bone formation. Therefore, the inventors have considered that, if a bone prosthetic material for sustained release of calcium ions is prepared, the function of promoting bone formation can be expected. Specifically, the inventors thought as follows: by incorporating calcium carbonate as a calcium source into the LASCol solid, sustained release of calcium ions in vivo and further enhancement of osteoinductivity could be expected. Under this hypothesis, the following test was conducted.

A columnar LASCol solid was prepared in a manner similar to that of Example 6, except that the final collagen concentration was 150 mg/mL and a solution containing 10 mM of calcium carbonate (CaCo₃) was used. As a template, a columnar template (diameter: 3.5 mm, length: 1 mm) was used. Here, the obtained LASCol solid is referred to as "CaCo₃-impregnated 150 mg/mL LASCol solid".

A 1 mm femur defect population was prepared in a manner similar to that of Example 10, except that a femur was resected by a width of 1 mm in each rat. Four individuals (n=4) of the CaCo₃-impregnated 150 mg/mL LASCol solid implanted population were prepared. After the rats were kept for a predetermined period of time, the bone regeneration status was evaluated.

14 days after implantation, computed tomography was carried out with a µCT device in a manner similar to that in [11-4. µCT image evaluation] to evaluate a degree of progress of bone adhesion. The result is shown in Fig. 24.

Fig. 24 shows a typical CT image in the CaCo₃-impregnated 150 mg/mL LASCol solid implanted population on the 14th day after implantation. As shown in Fig. 24, vigorous bone tissue formation was in progress on the 14th day after implantation. For example, in Fig. 24, it can be seen that bone regeneration is clearly promoted, as compared with Fig. 15 described above.

Based on those results, the CaCO₃-containing LASCol solid is expected to be clinically applied as an innovative bone prosthetic material for sustained release of calcium ions in a bone defect site, in addition to its osteoinductivity.

### [Example 14. bFGD-containing LASCol solid]

Fibroblast growth factors (hereinafter referred to as "bFGF") are known to promote bone regeneration by proliferating and differentiating undifferentiated mesenchymal stem cells. The LASCol solid begins to be degraded when the LASCol solid is embedded in a living body. Based on this fact, the inventors considered that the LASCol solid has a function as a bone prosthetic material which serves as a sustained release material which continues to release a physiologically active substance such as bFGF. Based on this idea, the inventors prepared a LASCol solid containing recombinant human basic fibroblast growth factors (product name: Fiblast, KAKEN PHARMACEUTICAL CO., LTD.) An effect of the LASCol solid containing basic fibroblast growth factors on bone regeneration was investigated using a critical femur deficiency rat model, which is generally considered not to show bone adhesion in the natural course.

A columnar LASCol solid was prepared in a manner similar to that of Example 6, except that a final collagen concentration was 100 mg/mL and a solution containing 12 µg of fibroblast growth factors (bFGF, product name: Fiblast, available from KAKEN PHARMACEUTICAL CO., LTD) was used. As a template, a columnar template (diameter: 3.5 mm, length: 4 mm) was used. Here, the obtained LASCol solid is referred to as "bFGF-impregnated 100 mg/mL LASCol solid".

A 4 mm femur defect population was prepared in a manner similar to that of Example 10. One individual (n=1) was prepared as a control (in which nothing was implanted into the bone defect site) and one individual (n=1) was prepared as the bFGF-impregnated 100 mg/mL LASCol solid implanted individual. After the rats were kept for a predetermined period of time, the bone regeneration status was evaluated.

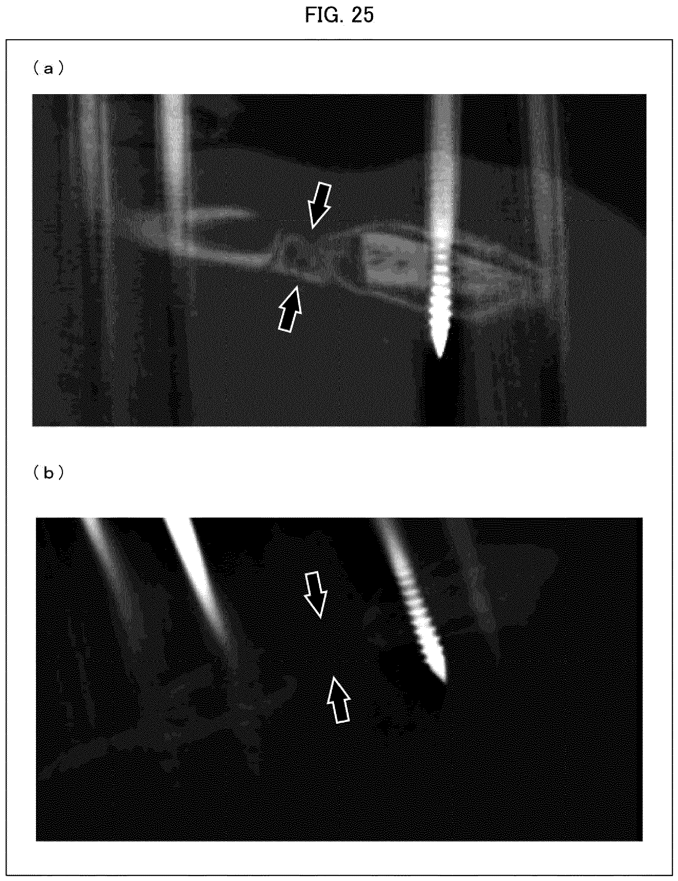

35 days after implantation, computed tomography was carried out with a µCT device in a manner similar to that in [11-4. µCT image evaluation] to evaluate a degree of progress of bone adhesion. The results are shown in Fig. 25.

Fig. 25 shows CT images of (a) the bFGF-impregnated 100 mg/mL LASCol solid implanted individual and (b) the 4 mm femur defect individual (control) taken 35 days after implantation. As shown in Fig. 25, bone regeneration was seen in the bFGF-impregnated 100 mg/mL LASCol solid implanted individual, whereas bone regeneration was not seen in the 4 mm femur defect individual.

As such, the results shown in Figs. 24 and 25 revealed that the CaCO₃-impregnated LASCol solid and the bFGF-impregnated LASCol solid are expected to be clinically applied as a novel bone regeneration material that does not require cell transplantation.

### Industrial Applicability

The present invention can be widely utilized in the field of materials (e.g., in the field of bone disease treatment, or in the field of cell culture). More specifically, the present invention can be widely used in treatment of fractures, bone tumors, and osteomyelitis, or in in vitro cell culture.

## Claims

1. A collagen solid, comprising a collagen-cysteine protease degradation product or an atelocollagen-cysteine protease degradation product,
said collagen solid having a density of 50 mg/cm³ or more.

2. The collagen solid as set forth in claim 1, wherein said collagen solid has a tangent modulus of 90 kPa or more.

3. The collagen solid as set forth in claim 1 or 2, further comprising an optional substance.

4. A biomaterial, comprising a collagen solid recited in any one of claims 1 through 3.

5. An ex vivo material, comprising a collagen solid recited in any one of claims 1 through 3.

6. A bone regeneration material, comprising a collagen solid recited in any one of claims 1 through 3.

7. A method for producing a collagen solid recited in any one of claims 1 through 3, said method comprising:
- a degradation step of degrading collagen or atelocollagen with a cysteine protease; and
- a removal step of removing a solvent from a collagen degradation product or an atelocollagen degradation product which has been obtained in the degradation step.

8. The method as set forth in claim 7, wherein, in the removal step, an optional substance is added to the collagen degradation product or the atelocollagen degradation product which has been obtained in the degradation step to obtain a mixture, and then the solvent is removed from the mixture.

9. The method as set forth in claim 7, wherein, in the removal step, a collagen solid which has been obtained in the removal step is caused to adsorb an optional substance.


SEQUENCE LISTING

<110>  KINKI UNIVERSITY
       KOBE UNIVERSITY

<120>  COLLAGEN SOLID MATERIAL, METHOD FOR PRODUCING COLLAGEN SOLID
MATERIAL, BIOMATERIAL, AND EX-VIVO MATERIAL

<130>  KI19330

<150>  JP 2018-232811
<151>  2018-12-12

<150>  JP 2019-081130
<151>  2019-04-22

<160>  3

<170>  PatentIn version 3.5

<210>  1
<211>  10
<212>  PRT
<213>  Artificial Sequence

<220>
<223>  Partial Sequence of Protein


<220>
<221>  misc_feature
<222>  (2)..(3)
<223>  Xaa can be any naturally occurring amino acid

<220>
<221>  misc_feature
<222>  (5)..(6)
<223>  Xaa can be any naturally occurring amino acid

<220>
<221>  misc_feature
<222>  (8)..(9)
<223>  Xaa can be any naturally occurring amino acid

<400>  1

Gly Xaa Xaa Gly Xaa Xaa Gly Xaa Xaa Gly
1               5                   10


<210>  2
<211>  22
<212>  PRT
<213>  Artificial Sequence

<220>
<223>  Partial Sequence of Protein


<220>
<221>  misc_feature
<222>  (2)..(3)
<223>  Xaa can be any naturally occurring amino acid

<220>
<221>  misc_feature
<222>  (5)..(6)
<223>  Xaa can be any naturally occurring amino acid

<220>
<221>  misc_feature
<222>  (8)..(9)
<223>  Xaa can be any naturally occurring amino acid

<220>
<221>  misc_feature
<222>  (11)..(12)
<223>  Xaa can be any naturally occurring amino acid

<220>
<221>  misc_feature
<222>  (14)..(15)
<223>  Xaa can be any naturally occurring amino acid

<220>
<221>  misc_feature
<222>  (17)..(18)
<223>  Xaa can be any naturally occurring amino acid

<220>
<221>  misc_feature
<222>  (20)..(21)
<223>  Xaa can be any naturally occurring amino acid

<400>  2

Gly Xaa Xaa Gly Xaa Xaa Gly Xaa Xaa Gly Xaa Xaa Gly Xaa Xaa Gly
1               5                   10                  15

Xaa Xaa Gly Xaa Xaa Gly
            20

<210>  3
<211>  13
<212>  PRT
<213>  Artificial Sequence

<220>
<223>  Partial Sequence of Protein

<220>
<221>  misc_feature
<222>  (1)..(3)
<223>  Xaa can be any naturally occurring amino acid

<220>
<221>  misc_feature
<222>  (5)..(6)
<223>  Xaa can be any naturally occurring amino acid

<220>
<221>  misc_feature
<222>  (8)..(9)

<223>  Xaa can be any naturally occurring amino acid

<220>
<221>  misc_feature
<222>  (11)..(12)
<223>  Xaa can be any naturally occurring amino acid

<400>  3

Xaa Xaa Xaa Gly Xaa Xaa Gly Xaa Xaa Gly Xaa Xaa Gly
1               5                   10